# EUROPEAN PATENT APPLICATION

(11) **EP 4 534 529 A1**
(43) Date of publication of application: **09.04.2025**
(21) Application number: 23815294.6
(22) Date of filing: 01.06.2023
(51) Int. Cl.: C07D 213/89

(54) **METHOD FOR PREPARING PYRIDINE OXYNITRIDE COMPOUND**

(30) Priority: 02.06.2022 CN 202210629774; 19.05.2023 CN 202310570708
(71) Applicant: Shanghai Jemincare Pharmaceutical Co., Ltd., Shanghai 201203 (CN)
(72) Inventor: ZHANG, Yong, Shanghai 201203 (CN); CAO, Cheng, Shanghai 201203 (CN); ZHANG, Fei, Shanghai 201203 (CN)
(74) Representative: J A Kemp LLP
(86) International application number: PCT/CN2023/097882
(87) International publication number: WO 2023/232117

(57) **Abstract**

The present disclosure discloses a method for preparing a pyridine nitrogen oxide compound; specifically, the present disclosure discloses a method for preparing a compound represented by formula (I), a pharmaceutically acceptable salt or hydrate thereof, wherein the compound represented by formula (I-5) or a pharmaceutically acceptable salt thereof comes into contact with the compound represented by formula (I-6) or a pharmaceutically acceptable salt thereof to obtain the compound represented by formula (I) or a pharmaceutically acceptable salt or hydrate thereof; the method is simple to operate, has a high yield, and is suitable for industrial mass production.

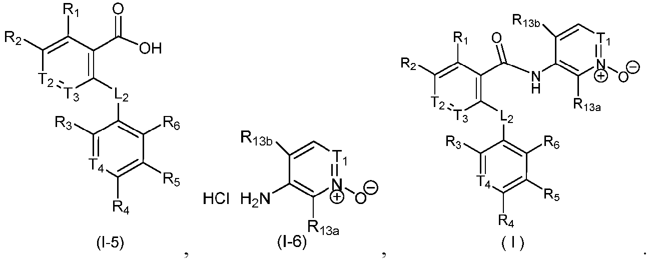

## Description

The present disclosure claims the following priorities:
1) CN202210629774.5, 02 June 2022,
2) CN202310570708.X, 19 May 2023.

### TECHNICAL FIELD

The present disclosure belongs to the field of medicinal chemistry; specifically, the present disclosure relates to a method for preparing a pyridine nitrogen oxide compound and a crystal form thereof.

### BACKGROUND ART

Pain is one of the most common clinical symptoms. It is the fifth vital sign after respiration, pulse, blood pressure, and body temperature and seriously affects the quality of life of patients. According to statistics, the global market for analgesics was approximately $36 billion in 2018 and is expected to reach $56 billion in 2023. Acute moderate and severe patients mainly depend on opioids, accounting for about two-thirds of the analgesic market share, which will grow steadily at a compound annual growth rate of 2.5% in the future. The number of chronic pain patients, dominated by neuropathic pain and arthritic pain, is increasing year by year and the market is expected to exhibit a compound annual growth rate of around 18%, which will be a major driving force for the continued growth of the global pain market over the next decade.

Recent research has progressively revealed that sodium channel subtype 1.8 (NaV1.8) plays an important role in the development and transmission of pain sensation. NaV1.8 is a voltage-gated sodium ion channel that is mainly expressed in afferent neurons, including sensory neurons. It plays an important role in maintaining the excitability of nociceptive sensory neurons, the release and persistence of action potentials, and the regulation of pain sensitivity by controlling the influx and outflow of sodium ions into and out of cells. Patients with NaV1.8 activated mutations develop small fiber neuropathy (which is mainly responsible for damage to pain-transmitted Aδ fibers and unmyelinated C-type fibers), resulting in paroxysmal pain. Chronic inflammation, diabetes, and other diseases can cause increased expression or property changes of NaV1.8, which sensitizes nociceptive neurons and causes a variety of pain. However, NaV1.8 gene knockout mice are not sensitive to pain.

Patent application CN112479996A (disclosure date 12 March 2021) discloses a novel NaV1.8 inhibitor having the general formula 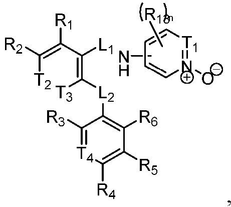 and also discloses the compound

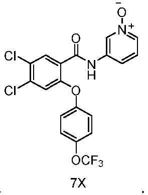

A patent application file having application number PCT/CN2022/080430 (application date 11 March 2022) discloses the crystal form A and crystal form B of the compound 7X.

### SUMMARY

The inventors found that the synthetic route of compound 7X in patent application CN112479996A is as follows:

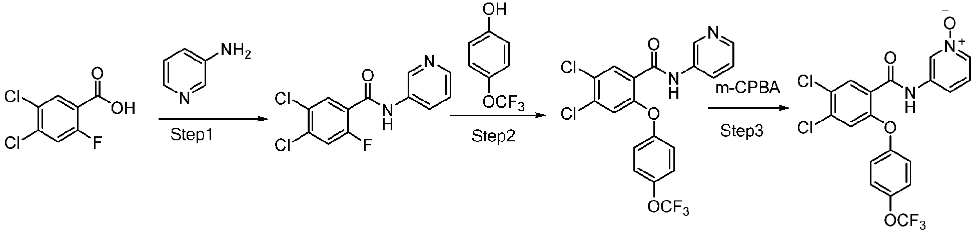

Step 2 in this route has a low yield, and step 3 uses m-CPBA as the oxidant, which has many impurities, which is not conducive to the quality control of the final compound as the drug substance and not suitable for industrial mass production.

On that basis, the inventors developed a new synthesis method that is more productive, less costly, simpler to operate, has higher product purity, complies with the quality standards of the drug substance, and is suitable for industrial scale production relative to the process of the prior art.

In a first aspect of the disclosure, the disclosure proposes a process for producing the compound represented by formula (I) or a pharmaceutically acceptable salt or hydrate thereof. According to the embodiments of the disclosure, the method comprises the compound represented by formula (I-5) or a pharmaceutically acceptable salt thereof coming into contact with the compound represented by formula (I-6) or a pharmaceutically acceptable salt thereof to obtain the compound represented by formula (I) or a pharmaceutically acceptable salt or hydrate thereof, 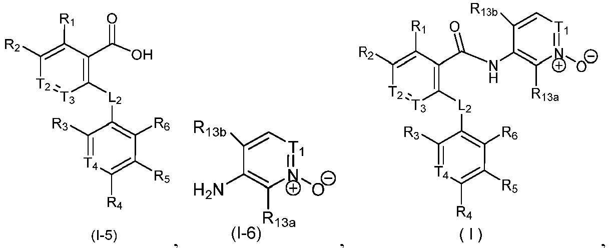
T₁ is selected from N or C(R₇);
T₂ is selected from N or C(R₈);
T₃ is selected from N or C(R₉);
T₄ is selected from N or C(R₁₀);
R₁, R₂, R₈, and R₉ are independently selected from H, halogen, OH, NH₂, CN, SF₅, C₁₋₆ alkyl, C₁₋₆ alkoxy, C₁₋₆ alkylamino, vinyl-C₁₋₆ alkyl-, C₃₋₆ cycloalkyl, 3-6 membered heterocycloalkyl, C₃₋₆ cycloalkyl-C₁₋₆ alkyl-, 3-6 membered heterocycloalkyl-C₁₋₆ alkyl-, 3-6 membered heterocycloalkyl-C₁₋₆ alkyl-, phenyl-C₁₋₃ alkyl-, C₃₋₆ cycloalkyl-C₁₋₃ alkyl-, 3-6 membered heterocycloalkyl-C₁₋₃ alkyl-, phenyl-C₁₋₃ alkyl-phenyl-C₁₋₃ alkyl-NH-, 5-6 membered heteroaryl-C₁₋₃ alkyl-, 5-6 membered heteroaryl-C₁₋₃ alkyl-O-, and 5-6 membered heteroaryl-C₁₋₃ alkyl-NH-, the C₁₋₆ alkyl, C₁₋₆ alkoxy, C₁₋₆ alkylamino, vinyl-C₁₋₆ alkyl-, C₃₋₆ cycloalkyl, 3-6 membered heterocycloalkyl, C₃₋₆ cycloalkyl-C₁₋₆ alkyl-, 3-6 membered heterocycloalkyl-C₁₋₆ alkyl-, 3-6 membered heterocycloalkyl-C₁₋₆ alkyl-, phenyl-C₁₋₃ alkyl-, C₃₋₆ cycloalkyl-C₁₋₃ alkyl-O-, 3-6 membered heterocycloalkyl-C₁₋₃ alkyl-O-, phenyl-C₁₋₃ alkyl-O-, phenyl-C₁₋₃ alkyl-NH-, 5-6 membered heteroaryl-C₁₋₃ alkyl-O-, or 5-6 membered heteroaryl-C₁₋₃ alkyl-NH- being optionally substituted with 1, 2, or 3 of R;
R₃, R₄, R₅, R₆, and R₁₀ are independently selected from H, halogen, OH, NH₂, SF₅, CN, C₁₋₆ alkyl, C₁₋₆ alkylamino, C₁₋₆ alkoxy, C₃₋₆ cycloalkyl, -O-C₃₋₆ cycloalkyl, 3-6 membered heterocycloalkyl, C₃₋₆ cycloalkyl-C₁₋₆ alkyl-, and 3-6 membered heterocycloalkyl-C₁₋₆ alkyl-, respectively, the C₁₋₆ alkyl, C₁₋₆ alkylamino, C₁₋₆ alkoxy, C₃₋₆ cycloalkyl, -O-C₃₋₆ cycloalkyl, 3-6 membered heterocycloalkyl C₃₋₆ cycloalkyl-C₁₋₆ alkyl, -or 3-6 membered heterocycloalkyl-C₁₋₆ alkyl- being optionally substituted with 1, 2, or 3 of R;
R₇ is selected from H, F, Cl, Br, I, C₁₋₆ alkyl, C₁₋₆ alkoxy, and C₁₋₆ alkylamino, the C₁₋₆ alkyl, C₁₋₆ alkoxy, or C₁₋₆ alkylamino being optionally substituted with 1, 2, or 3 of R;
L₂ is selected from O, S, NH, and CH₂, the CH₂ being optionally substituted with 1 or 2 of R, and NH being optionally substituted with R;
R₁₃ₐ and R_{13b} are independently selected from H, halogen, and C₁₋₆ alkyl, the C₁₋₆ alkyl being optionally substituted with 1, 2, or 3 of R;
R is independently selected from H, D halogen, OH, NH₂, CN, 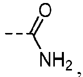 C₁₋₆ alkyl, C₁₋₆ alkoxy, C₁₋₆ alkylthio, and C₁₋₆ alkylamino, the C₁₋₆ alkyl, C₁₋₆ alkoxy, C₁₋₆ alkylthio, or C₁₋₆ alkylamino being optionally substituted with 1, 2, or 3 of R';
R' is selected from F, Cl, Br, I, OH, NH₂, and CH₃;
the above 3-6 membered heterocycloalkyl or 5-6 membered heteroaryl comprises 1, 2, or 3 heteroatoms or heteroatomic groups independently selected from -O-, -NH-, -S-, -C(=O)-, -C(=O)O-, -S(=O)-, -S(=O)₂-, and N.

In some schemes of the present disclosure, the above R is selected from H, D, F, Cl, Br, I, OH, NH₂, 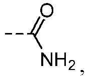 Me, CF₃, CHF₂, CH₂F, 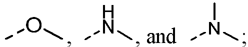 the remaining variables are defined in the present disclosure.

In some schemes of the present disclosure, the above R₁, R₂, R₈, and R₉ are independently selected from H, halogen, OH, NH₂, CN, SF₅, C₁₋₃ alkyl, C₁₋₃ alkoxy, C₁₋₃ alkylamino, vinyl-C₁₋₃ alkyl-, C₃₋₆ cycloalkyl, 3-6 membered heterocyclic alkyl, C₃₋₆ cycloalkyl-C₁₋₃ alkyl-, 3-6 membered heterocyclic alkyl-C₁₋₃ alkyl-, 3-6 membered heterocyclic alkyl-C₁₋₃ alkyl-, phenyl-C₁₋₃ alkyl-, phenyl-C₁₋₃ alkyl-, phenyl-C₁₋₃ alkyl-pyridinyl-C₁₋₃ alkyl-, pyrimidyl-C₁₋₃ alkyl-, thiophenyl-C₁₋₃ alkyl-, thiazolyl-C₁₋₃ alkyl-, pyrazol-C₁₋₃ alkyl-, imidazolyl-C₁₋₃ alkyl-, pyridinyl-C₁₋₃ alkyl-O-, pyrimidyl-C₁₋₃ alkyl-O-, thiophenyl-C₁₋₃ alkyl-O-, thiazolyl-C₁₋₃ alkyl-O-, pyrazol-C₁₋₃ alkyl-O-, imidazolyl-C₁₋₃ alkyl-O-, pyridinyl-C₁₋₃ alkyl-NH-, pyrimidyl-C₁₋₃ alkyl-NH-, thiophenyl-C₁₋₃ alkyl-NH-thiazolyl-C₁₋₃ alkyl-NH-, pyrazoyl-C₁₋₃ alkyl-NH-, and imidazolyl-C₁₋₃ alkyl-NH-, the C₁₋₃ alkyl, C₁₋₃ alkoxy, C₁₋₃ alkylamino, vinyl-C₁₋₃ alkyl-, C₃₋₆ cycloalkyl, 3-6 membered heterocycloalkyl, C₃₋₆ cycloalkyl-C₁₋₃ alkyl-, 3-6 membered heterocycloalkyl-C₁₋₃ alkyl-, 3-6 membered heterocycloalkyl-C₁₋₃ alkyl-, phenyl-C₁₋₃ alkyl-, phenyl-C₁₋₃ alkyl-, phenyl-C₁₋₃ alkyl-pyrimidine-C₁₋₃ alkyl-, thienyl-C₁₋₃ alkyl-, thiazolyl-C₁₋₃ alkyl-, pyrazol-C₁₋₃ alkyl-, imidazolyl-C₁₋₃ alkyl-, pyridyl-C₁₋₃ alkyl-O-, pyrimidine-C₁₋₃ alkyl-O-, thienyl-C₁₋₃ alkyl-O-, thiazolyl-C₁₋₃ alkyl-O-, pyrazol-C₁₋₃ alkyl-O-, imidazolyl-C₁₋₃ alkyl-O-, pyridyl-C₁₋₃ alkyl-NH-, pyrimidine-C₁₋₃ alkyl-NH-, thienyl-C₁₋₃ alkyl-NH-, thiazolyl-C₁₋₃ alkyl-NH-pyrazoyl-C₁₋₃ alkyl-NH- or imidazolyl-C₁₋₃ alkyl-NH- being optionally substituted with 1, 2, or 3 of R; the remaining variables are defined in the present disclosure.

In some schemes of the present disclosure, R₁, R₂, R₈, and R₉ are independently selected from H, F, Cl, Br, I, OH, NH₂, CN, SF₅, Me, CF₃, CHF₂, CH₂F, CF₃CF₂, OCF₃, HOCH₂CH₂O, CH₃NHCH₂CH₂O, (CH₃)₂NCH₂CH₂O 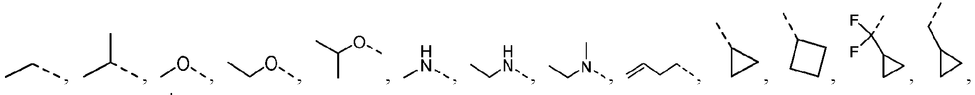 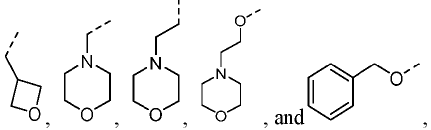 and the remaining variables are defined in the present disclosure.

In some schemes of the present disclosure, the above structural unit 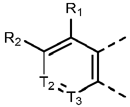 is selected from 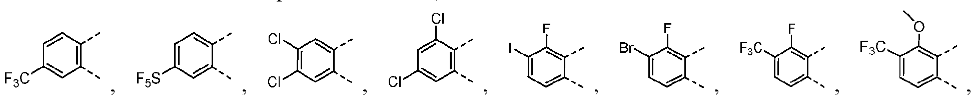 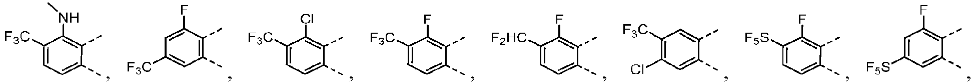 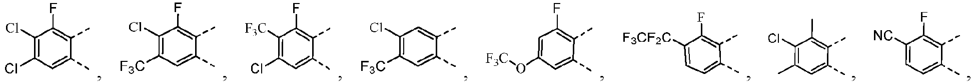 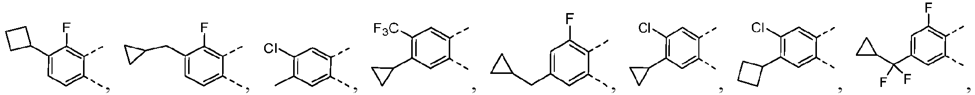 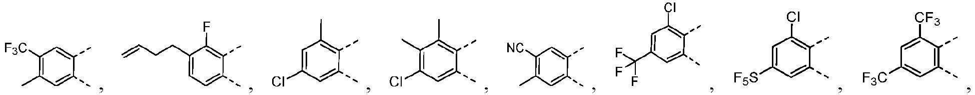 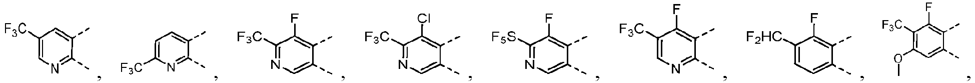 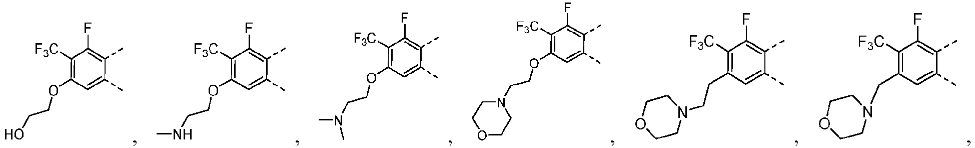 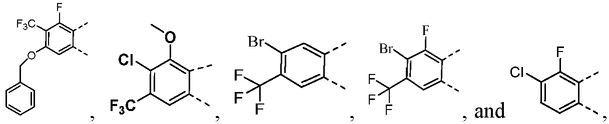 and the remaining variables are defined in the present disclosure.

In some schemes of the present disclosure, the above R₃, R₄, R₅, R₆, and R₁₀ are independently selected from H, halogen, OH, NH₂, SF₅, CN, C₁₋₃ alkyl, C₁₋₃ alkylamino, C₁₋₃ alkoxy, C₃₋₆ cycloalkyl, -O-C₃₋₆ cycloalkyl, 3-6 membered heterocycloalkyl, C₃₋₆ cycloalkyl-C₁₋₃ alkyl, and 3-6 membered heterocycloalkyl-C₁₋₃ alkyl, the C₁₋₃ alkyl, C₁₋₃ alkylamino, C₁₋₃ alkoxy, C₃₋₆ cycloalkyl, -O-C₃₋₆ cycloalkyl, 3-6 membered heterocycloalkyl C₃₋₆ cycloalkyl-C₁₋₃ alkyl, -or 3-6 membered heterocycloalkyl-C₁₋₃ alkyl- being optionally substituted with 1, 2, or 3 of R, and the remaining variables are defined in the present disclosure.

In some schemes of the present disclosure, R₃, R₄, R₅, R₆, and R₁₀ are independently selected from H, F, Cl, Br, I, OH, NH₂, SF₅, Me, CF₃, CHF₂, CH₂F, CN, CH(F₂)CH₃, CD₃, OCD₃, 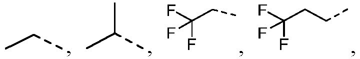 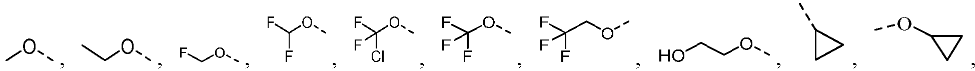 and 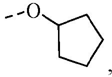 and the remaining variables are defined in the present disclosure.

In some schemes of the present disclosure, the above structural unit 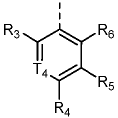 is selected from 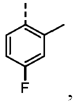 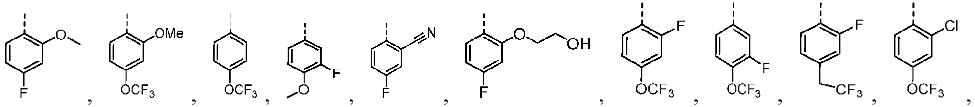 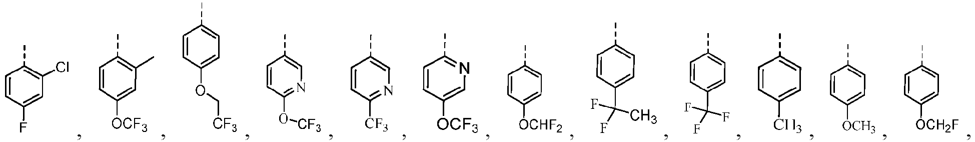 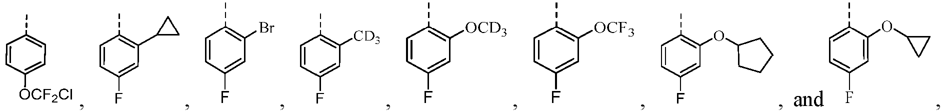 and the remaining variables are defined in the present disclosure.

In some schemes of the present disclosure, the above R₁₃ₐ and R_{13b} are independently selected from H, F, Cl, Br, I, and Me, and the remaining variables are defined in the present disclosure.

In some embodiments of the present disclosure, the above compound represented by formula (I) or its optical isomers are selected from: 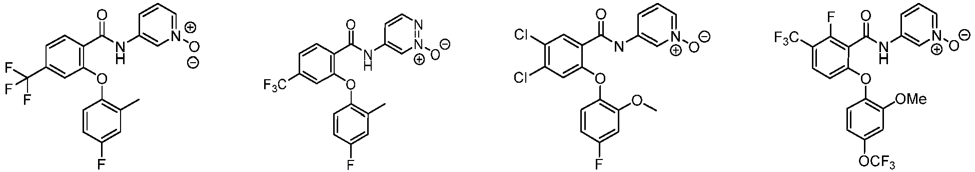 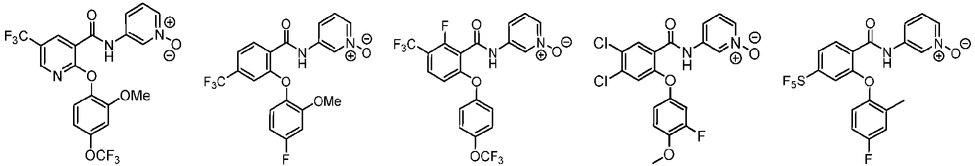 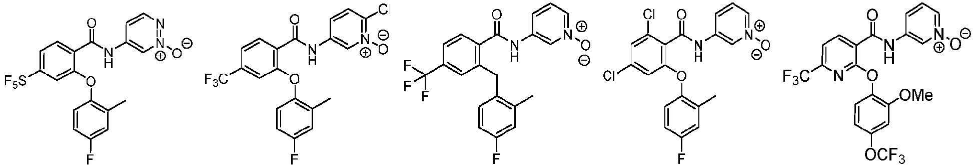 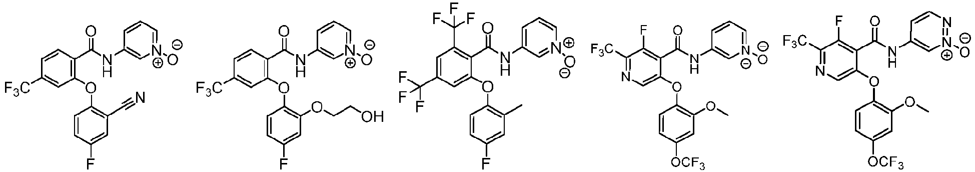 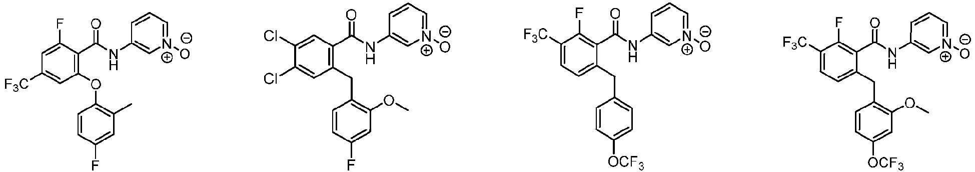 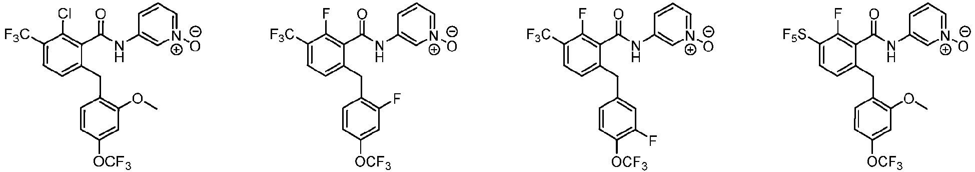 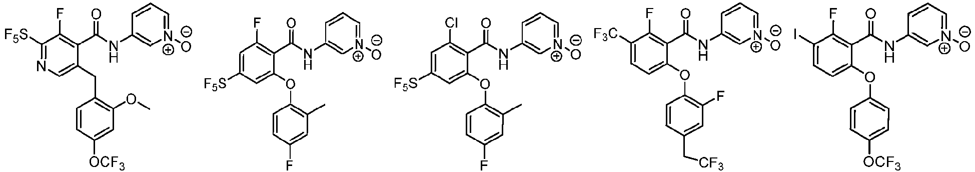 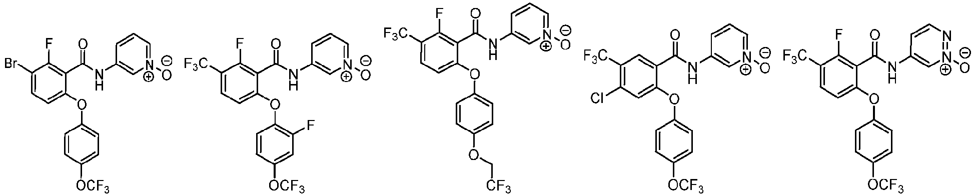 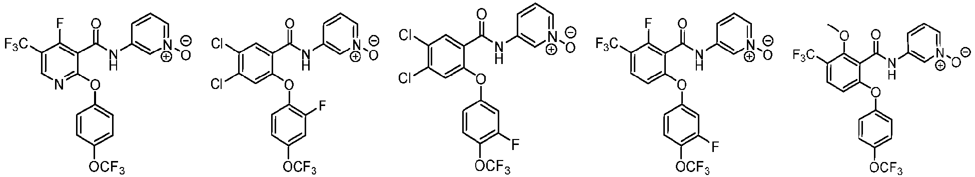 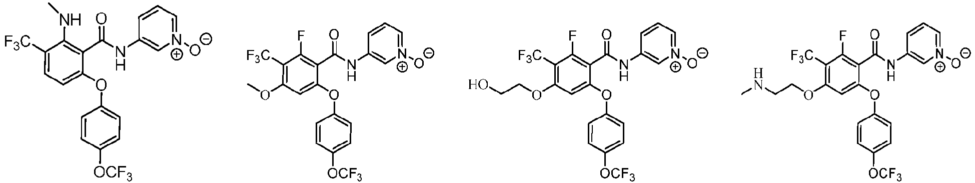 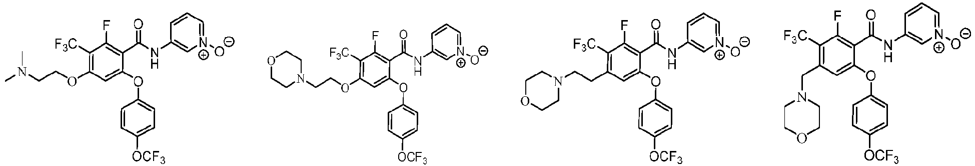 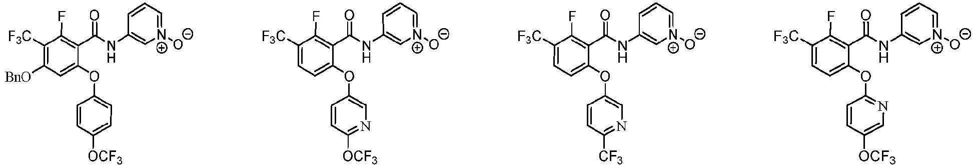 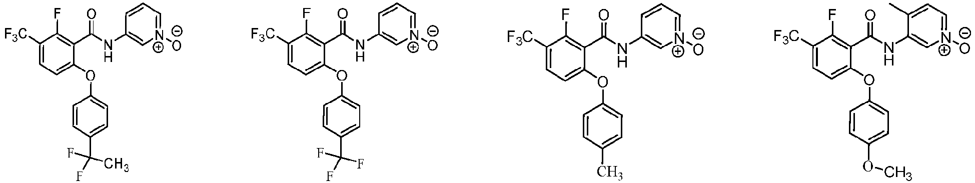 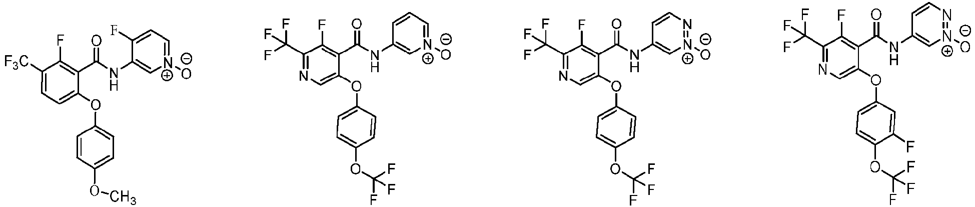 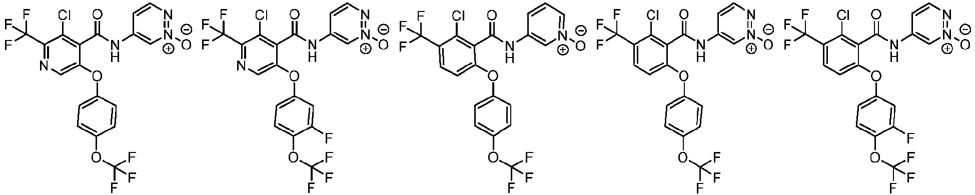 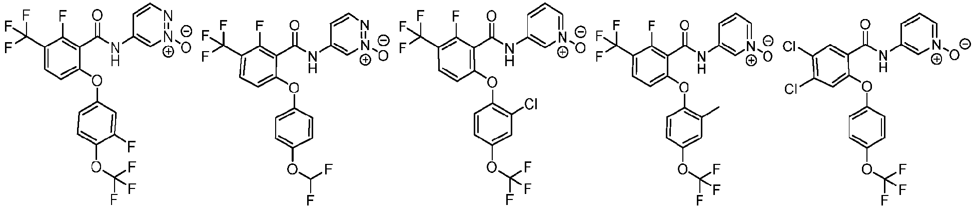 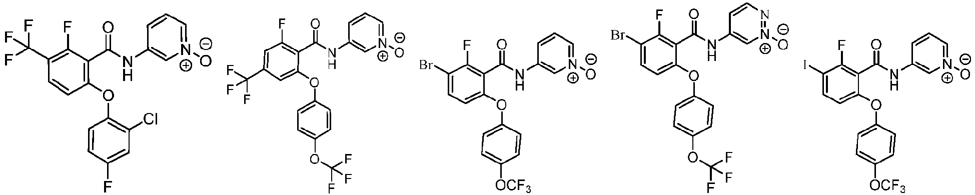 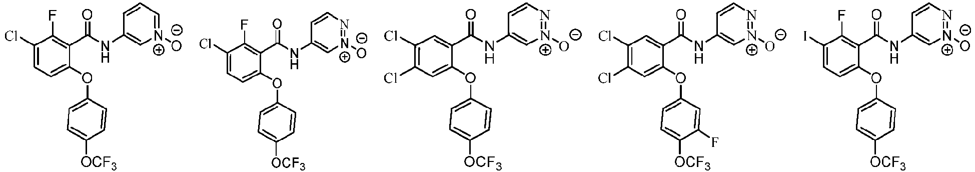 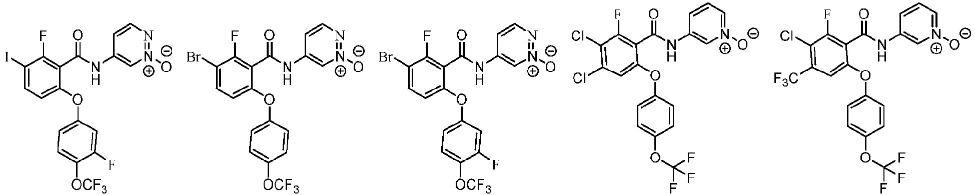 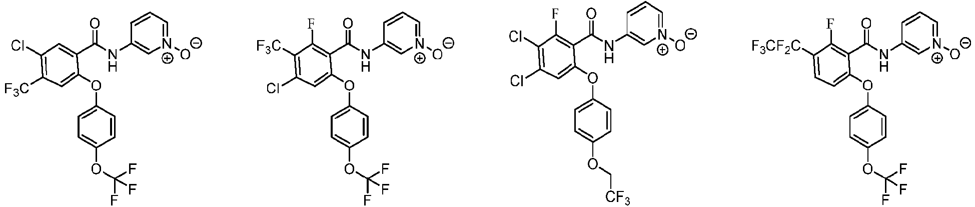 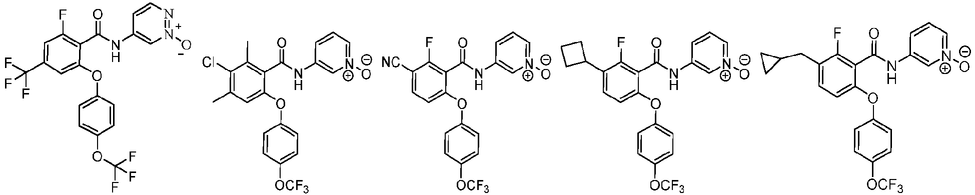 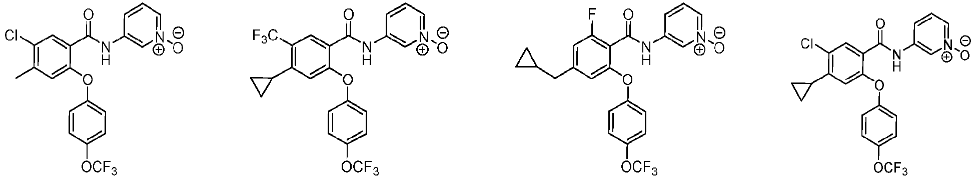 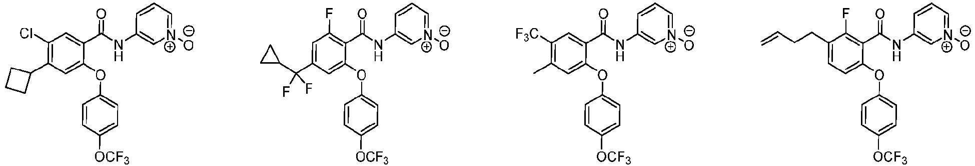 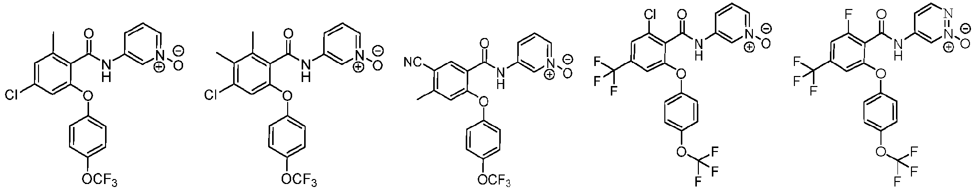 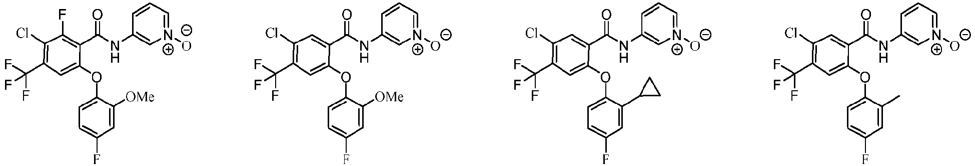 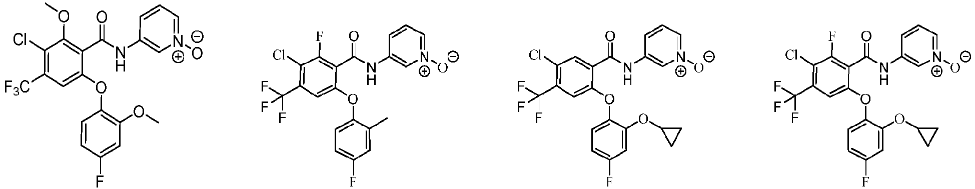 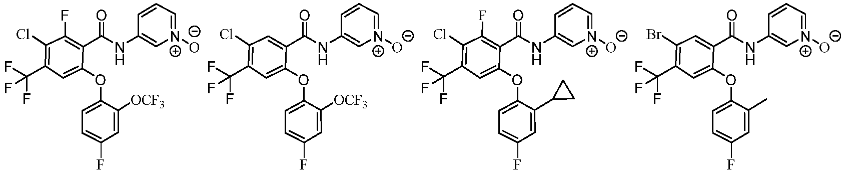 and

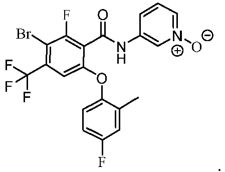

In some schemes of the present disclosure, the above compounds represented by formula (I) are selected from 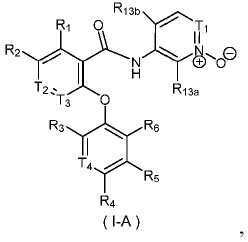 and the remaining variables are defined by the present disclosure.

In some schemes of the present disclosure, the above compounds represented by formula (I) are selected from 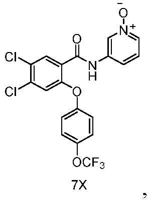 and the remaining variables are defined by the present disclosure.

In some schemes of the present disclosure, the above compounds represented by formula (I) are selected from 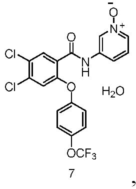 and the remaining variables are defined by the present disclosure.

In some schemes of the present disclosure the above compounds represented by formula (I-5) are selected from 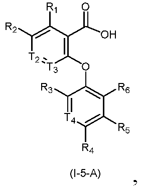 and the remaining variables are defined by the present disclosure.

In some schemes of the present disclosure, the above compounds represented by formula (I-5) are selected from 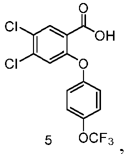 and the remaining variables are defined by the present disclosure.

In some schemes of the present disclosure, the above compounds represented by formula (I-6) are selected from 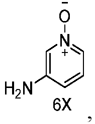 and the remaining variables are defined by the present disclosure.

In some schemes of the present disclosure, the above compounds represented by formula (I-6) are selected from 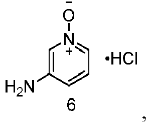 and the remaining variables are defined by the present disclosure.

In some schemes of the present disclosure, the contact described above is carried out under the conditions of a condensation agent, and the remaining variables are defined in the present disclosure.

In some schemes of the present disclosure, the condensation agent is selected from 2-(7-azabenzotriazole)-N,N,N',N'-tetramethyluronium hexafluorophosphate, 1-(3-dimethylaminopropyl)-3-ethylcarbodiimide hydrochloride/1-hydroxybenzotriazole, and N,N'-carbonyldiimidazole, and the remaining variables are defined in the present disclosure. The inventors found that condensation works well in the presence of these condensation agents.

In some schemes of the present disclosure, the above condensation agent is 2-(7-azabenzotriazole)-N,N,N',N'-tetramethyluronium hexafluorophosphate, and the remaining variables are defined in the present disclosure. The inventors found that condensation works well in the presence of these condensation agents.

In some schemes of the present disclosure, the above condensation agent is one of N,N,N',N'-tetramethylchloroformamidine hexafluorophosphate or N-methylimidazole, and the remaining variables are defined in the present disclosure.

In some schemes of the present disclosure, the above condensation agent is a mixture of N,N,N',N'-tetramethylchloroformamidine hexafluorophosphate and N-methylimidazole, and the remaining variables are defined in the present disclosure.

In some schemes of the present disclosure, the molar ratio of the compound represented by formula (I-5) to the 2-(7-azabenzotriazole)-N,N,N',N'-tetramethyluronium hexafluorophosphate is 1:(1.0-2.0), and the remaining variables are defined in the present disclosure. The inventors found that condensation works well at these ratios.

In some schemes of the present disclosure, the molar ratio of the compound represented by formula (I-5) to the 2-(7-azabenzotriazole)-N,N,N',N'-tetramethyluronium hexafluorophosphate is 1:(1.2-1.5), and the remaining variables are defined in the present disclosure. The inventors found that condensation works better at these ratios.

In some schemes of the present disclosure, the molar ratio of the compound represented by formula (I-5) to the N,N,N',N'-tetramethylchloroformamidine hexafluorophosphate is 1:(1.0-2.0), and the remaining variables are defined in the present disclosure.

In some schemes of the present disclosure, the molar ratio of the compound represented by formula (I-5) to the N,N,N',N'-tetramethylchloroformamidine hexafluorophosphate is 1:(1.1-1.2), and the remaining variables are defined in the present disclosure.

In some schemes of the present disclosure, the molar ratio of the N-methylimidazole to the N,N,N',N'-tetramethylchloroformamidine hexafluorophosphate is 1: (1.2-2.0), and the remaining variables are defined in the present disclosure.

In some schemes of the present disclosure, the molar ratio of the N-methylimidazole to the N,N,N',N'-tetramethylchloroformamidine hexafluorophosphate is 1: (1.4-1.6), and the remaining variables are defined in the present disclosure.

In some schemes of the present disclosure, the described contact is carried out under alkaline conditions, and the remaining variables are defined in the present disclosure.

In some schemes of the disclosure, the above basic conditions are provided by N,N-diisopropylethylamine, and the remaining variables are defined in the present disclosure.

In some schemes of the present disclosure, the molar ratio of the compound represented by formula (I-5) to the N,N-diisopropylethylamine is 1:(1.0-5.0), and the remaining variables are defined in the present disclosure.

In some schemes of the present disclosure, the molar ratio of the compound represented by formula (I-5) to the N,N-diisopropylethylamine is 1:(2.5-3.0), and the remaining variables are defined in the present disclosure.

In some schemes of the present disclosure, the described contact is carried out under the condition that the solvent is N,N-dimethylformamide, acetonitrile, or dichloromethane, and the remaining variables are defined in the present disclosure.

In some schemes of the present disclosure, the described contact is carried out under the condition that the solvent is N,N-dimethylformamide, and the remaining variables are defined in the present disclosure.

In some schemes of the present disclosure, the compound represented by formula (I-5-A) is obtained by a substitution reaction between the compound represented by formula (I-2) and the compound represented by formula (I-3), 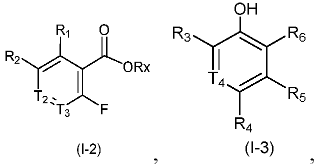 wherein Rx is selected from C₁₋₆ alkyl, and the remaining variables are defined in the present disclosure.

In some schemes of the present disclosure, the above compound represented by formula (I-2) or its optical isomers is selected from 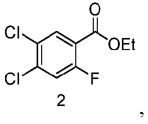 and the remaining variables are defined in the present disclosure.

In some schemes of the present disclosure, the above compound represented by formula (I-3) or its optical isomers is selected from 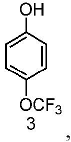 and the remaining variables are defined in the present disclosure.

In some schemes of the present disclosure, the above substitution reactions are carried out in the presence of an alkali, and the remaining variables are defined in the present disclosure.

In some schemes of the present disclosure, the above alkali is selected from Cs₂CO₃, K₂CO₃, and K₃PO₄, and the remaining variables are defined in the present disclosure. The inventors found that using the described alkalis produces a high yield.

In some schemes of the present disclosure, the above alkali is Cs₂CO₃, and the remaining variables are defined in the present disclosure. The inventors found that using Cs₂CO₃ produces a higher yield.

In some schemes of the present disclosure, the molar ratio of the compound represented by formula (I-2) to the Cs₂CO₃ is 1:(1.3-1.5), and the remaining variables are defined in the present disclosure.

In some schemes of the present disclosure, the above substitution reactions are carried out in a solvent of N,N-dimethylformamide or tetrahydrofuran, and the remaining variables are defined in the present disclosure.

In some schemes of the present disclosure, the molar ratio of the compound represented by formula (I-2) to the compound represented by formula (I-3) is 1:(1.10-1.25), and the remaining variables are defined in the present disclosure.

In some schemes of the present disclosure, the molar ratio of the compound represented by formula (I-2) to the compound represented by formula (I-3) is 1:1.15, and the remaining variables are defined in the present disclosure.

In some schemes of the present disclosure, the compound represented by formula (I-2) is obtained by esterification of the compound represented by formula (I-1), 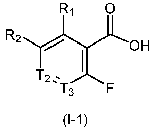 , and the remaining variables are defined in the present disclosure.

In some embodiments of the present disclosure, the compound represented by formula (I-1) or its optical isomer is selected from 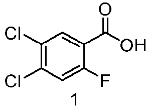 , and the remaining variables are defined in the present disclosure.

In some schemes of the present disclosure, the above esterification reaction is carried out in the presence of SOCl₂, and the remaining variables are defined in the present disclosure.

In some schemes of the present disclosure, the molar ratio of the compound represented by formula (I-1) to SOCl₂ is 1:(1.0-2.0), and the remaining variables are defined in the present disclosure.

In some schemes of the present disclosure, the molar ratio of the compound represented by formula (I-1) to SOCl₂ is 1:(1.2-1.5), and the remaining variables are defined in the present disclosure.

In some schemes of the present disclosure, the above esterification reaction is carried out under the condition that the solvent is ethanol, and the remaining variables are defined in the present disclosure.

In some schemes of the present disclosure, the ratio of the compound represented by formula (I-1) to the ethanol is (1:1672-2090). It should be noted that the ratio here refers to the molar volume ratio (mol:mL), and the remaining variables are defined in the present disclosure.

In some schemes of the present disclosure, the above esterification reaction further comprises post-treatment, and the remaining variables are defined in the present disclosure.

In some schemes of the present disclosure, the described post-treatment is carried out by adding an aqueous solution of sodium bicarbonate, and the remaining variables are defined in the present disclosure.

In another aspect of the disclosure, the disclosure also proposes an intermediate compound or a pharmaceutically acceptable salt thereof. According to an embodiment of the present disclosure, the structure of the intermediate compound described above is represented by compound 5,

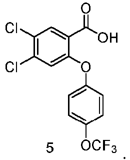

In yet another aspect of the present disclosure, the present disclosure also proposes the use of the compound represented by compound 5 or a pharmaceutically acceptable salt thereof in the preparation of the compound represented by compound 7X or its optical isomers.

In yet another aspect of the disclosure, the disclosure also proposes 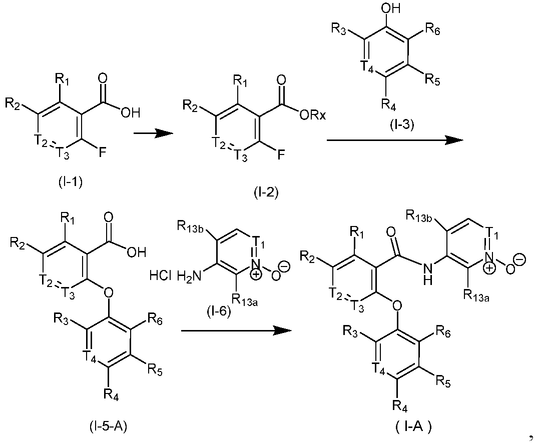 and the remaining variables are defined in the present disclosure.

In yet another aspect of the disclosure, the disclosure also proposes

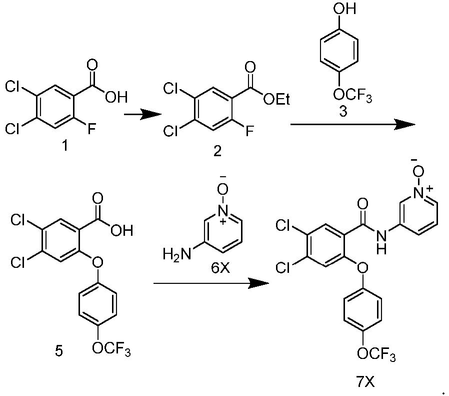

In yet another aspect of the disclosure, the disclosure also proposes

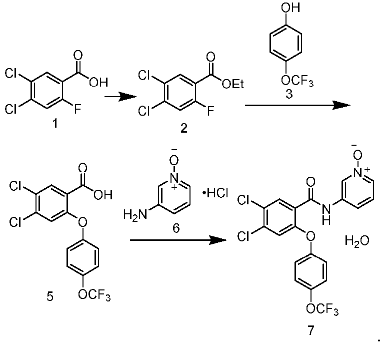

In yet another aspect of the disclosure, the disclosure proposes a method for preparing compound 1. According to an embodiment of the present disclosure, the method comprises performing a chlorination reaction on the compound represented by formula (X-1) to obtain compound 1, the chlorination reaction being carried out in one or more solvents of concentrated sulfuric acid, methanesulfonic acid, trifluoroacetic acid, phosphoric acid, or ethanesulfonic acid,

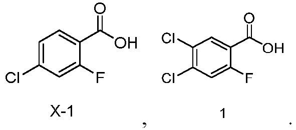

In yet another aspect of the disclosure, the disclosure proposes a method for preparing compound 1. According to an embodiment of the present disclosure, the method comprises performing a chlorination reaction on the compound represented by formula (X-1) to obtain compound 1, the chlorination reaction being carried out in a solvent of concentrated sulfuric acid

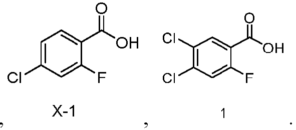

The inventor found that the method for preparing compound 1 in the prior art is the use of a Friedel-Crafts alkylation reaction, in which highly toxic carbon tetrachloride is used, and the reaction and post-treatment processes are relatively complex and produce a low yield. However, the method of the present disclosure is easy to perform, does not use highly toxic reagents, and has a high yield. Moreover, the chlorination reaction of the inventor's disclosure needs to be carried out under concentrated sulfuric acid conditions and under other solvent conditions, does not react, or produces a low yield.

In some schemes of the present disclosure, the above chlorination reactions are carried out under chlorinated reagent conditions, and the remaining variables are defined in the present disclosure.

In some schemes of the present disclosure, the above chlorinated reagents are selected from 1,3-dichloro-5,5-dimethylhydantoin and N-chlorosuccinimide, and the remaining variables are defined in the present disclosure.

In some schemes of the present disclosure, the molar ratio of the compound represented by the above formula (X-1) to 1,3-dichloro-5,5-dimethylhydantoin is 1:(0.5-0.7), and the remaining variables are defined in the present disclosure. The inventor found that the yield of the chlorination reaction was higher at the described ratio.

In some schemes of the present disclosure, the molar ratio of the compound represented by the above formula (X-1) to N-chlorosuccinimide is 1:(1.1-1.6), and the remaining variables are defined in the present disclosure. The inventor found that the yield of the chlorination reaction was higher at the described ratio.

In some schemes of the present disclosure, the chlorination reaction described above is carried out at a temperature of 55-60°C, and the remaining variables are defined in the present disclosure. The inventor found that within this temperature range, the yield was higher.

In another aspect of the disclosure, the disclosure also proposes a method for preparing compound 6. According to an embodiment of the present disclosure, the method comprises performing an oxidation reaction and a deprotection reaction on the compound represented by formula (Y-2) to obtain compound 6,

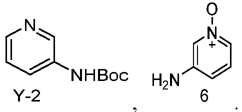

The inventors found that the method for preparing compound 6 in the prior art is to use nicotinic acid N-oxide as the starting material, use DPPA and tert-butanol to form a -Boc protected amino group, and remove the -Boc protection to obtain the target product. In this method, a higher-cost DPPA is used and too many impurities are generated during the reaction process, or the compound represented by formula (Y-2) is directly oxidized by pyridine using MCPBA. With that method it is extremely easy to produce a polymerization product of two molecular products with low yield. The method proposed by the present disclosure is simple to operate, uses cheap starting materials, and post-treatment is convenient, making it suitable for industrial production.

In some schemes of the present disclosure, the compound represented by formula (Y-2) is obtained by performing an acylation reaction with the compound represented by formula (Y-1), 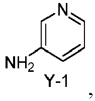 and the remaining variables are defined in the present disclosure.

### Definitions and Description

The following terms and phrases used herein are intended to have the following meanings, unless otherwise stated. A particular term or phrase should not be considered uncertain or unclear if it is not specifically defined, but rather should be understood in its ordinary sense. When trade names appear in this text, they are meant to refer to their corresponding products or active ingredients thereof.

The term "contact" should be understood in broad terms, which may be any means of enabling at least two reactants to chemically react; for example, it may be the mixing of the two reactants under appropriate conditions. As required, the reactants that need to be in contact can be mixed by stirring, whereby, the type of stirring is not particularly restricted, e.g., it may be mechanical, i.e., stirring is performed by the action of mechanical forces.

The compounds of the present disclosure may exist in specific geometric or stereoisomeric forms. The present disclosure envisions that all such compounds, including cis and trans isomers, (-)-and (+)-enantiomers, (R)-and (S)-enantiomers, diastereomers, (D)-isomers, (L)-isomers, and their racemic mixtures and other mixtures, such as enantiomer- or diastereomer-enriched mixtures, all fall within the scope of the present disclosure. Additional asymmetric carbon atoms may be present in substituents such as alkyl. All of these isomers, as well as their mixtures, are included within the scope of the present disclosure.

There may be specific compounds of the present disclosure. Unless otherwise stated, the term "tautomer" or "tautomer form" refers to the fact that at room temperature, the different functional group isomers are in dynamic equilibrium and can rapidly interconvert. If the tautomer is possible (as in a solution), then the chemical equilibrium of the tautomer can be reached. For example, the proton tautomer (also known as prototropic tautomer) comprises interconversion via proton migration, such as keto-enol isomerization and imine-enamine isomerization. Valence tautomers comprise the reorganization of some of the bond-forming electrons to undergo interconversion. A specific example of keto-enol tautomerization is the tautomerization between the two tautomers pentan-2,4-dione and 4-hydroxypentan-3-en-2-one.

The compounds of the present disclosure may comprise an unnatural proportion of atomic isotopes on one or more of the atoms constituting the compound. For example, compounds can be labeled with a radio isotope, such as tritium (³H), iodine-125 (¹²⁵I), or C-14 (¹⁴C). For example, hydrogen can be replaced by heavy hydrogen to form a deuterated drug. The bond between deuterium and carbon is stronger than the bond between ordinary hydrogen and carbon. Compared to non-deuterated drugs, deuterated drugs have the advantages of reducing toxic side effects, increasing drug stability, enhancing therapeutic efficacy, and extending the biological half-life of the drug. The transformations of all isotopic compositions of the compounds of the present disclosure, whether radioactive or not, are included in the scope of the present disclosure. "Optional" or "optionally" refers to events or conditions that may, but not necessarily, occur as subsequently described, and such description includes the circumstances in which the event or condition occurs as well as the circumstances in which the event or condition does not occur.

When any variable, such as R, appears more than once in the composition or structure of a compound, its definition in each case is independent. Thus, if, for example, one group is substituted with 0-2 of R, the groups may optionally be substituted with at most two R, and there are independent options for R in each case. In addition, combinations of substituents and/or variants thereof are only permitted when such combinations are stable compounds. For example, 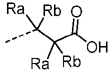 may be selected from 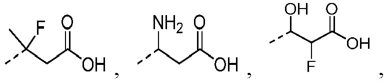 and 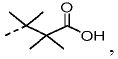 etc.

In the present disclosure, "room temperature" refers to a temperature ranging from about 10°C to about 40°C. In some embodiments, "room temperature" refers to a temperature ranging from about 20°C to about 30°C. In other embodiments, "room temperature" refers to 20°C, 22.5°C, 25°C, 27.5°C, etc.

In the present disclosure, "under room temperature or heating conditions" refers to the reaction occurring at a certain temperature, which is room temperature or a specific temperature reached by heating. For example, the reaction of preparing a compound of formula (III) from a compound of formula (IV) described in the present disclosure being carried out at room temperature or under heating conditions indicates that the reaction is carried out under certain temperature conditions, said certain temperature being room temperature or a specific temperature reached by heating; for example, the reaction being carried out under conditions of room temperature (such as 20°C-30°C) or heating to 30°C-65°C means that the reaction is carried out at 20°C-65°C.

In the context of the present disclosure, all figures disclosed herein are approximate values. The value of each number may vary by 1%, 2%, 5%, 7%, 8%, or 10%. Whenever a number with an N-value is disclosed, any number having a value within N+/-1%, N+/-2%, N+/-3%, N+/-5%, N+/-7%, N+/-8%, or N+/-10% is explicitly disclosed, where "+/-" refers to addition or subtraction. Whenever a lower limit, DL, and an upper limit, DU, of a range of values are disclosed, any values within the disclosed range are explicitly disclosed.

All the reaction steps described in the present disclosure react to a certain extent, such as starting material consumption of approximately more than 70%, more than 80%, more than 90%, or more than 95%, or after testing that the reaction starting material has been consumed, post-treatment is performed, such as cooling, collecting, extracting, filtering, separating, purifying, or a combination thereof. The degree of reaction can be detected by conventional methods, such as thin layer chromatography (TLC), high-performance liquid chromatography (HPLC), and gas chromatography (GC). Conventional methods can be used to post-treat the reaction solution, for example, by performing vacuum evaporation or conventional distillation of the reaction solvent, then collecting the crude product and directly putting it into the next step of the reaction; or directly filtering to obtain the crude product and directly putting it into the next reaction; or, after letting stand, pouring out the supernatant to obtain the crude product and directly putting it into the next reaction; or selecting appropriate organic solvents or combinations thereof for purification steps such as extraction, distillation, crystallization, column chromatography, washing, and slurrying.

The numerical values of the present disclosure, regardless of whether or not they are modified by the term "approximately", should be understood to be modified by "approximately", the term "approximately" being used to modify a numerical value differing by 10% from high to low. In some embodiments, "approximately" is used to modify a value that differs by 5% from high to low. In some embodiments, "approximately" is used to modify a value that differs by 3% or 2% or 1% from high to low. It is understood that the "approximate" modification of the numerical error range depends on the actual or reasonable error range of the modified numerical value.

During each step of the reaction process described in the present disclosure, reaction starting materials or other reagents can be added to the reaction system in a dropwise manner. The dropwise addition process and each step of the reaction are carried out under certain temperature conditions, and any temperature suitable for use in each dropwise addition process or reaction process is included in the present disclosure. In addition, many similar modifications or equivalent substitutions in the art, or equivalent temperatures and temperature ranges described in the present disclosure, are considered to be included in the scope of the disclosure. The present disclosure provides a preferred temperature or temperature range for each dropwise addition process, as well as a preferred reaction temperature for each reaction.

The solvents used in each reaction step described in the present disclosure are not particularly limited, and any solvent that can dissolve the starting material to a certain extent while not inhibiting the reaction is included in the present disclosure. In addition, many similar modifications or equivalent substitutions in the art, or equivalents to the solvents, solvent combinations, and different proportions of solvent combinations described in the present disclosure, are considered to be within the scope of the present disclosure. The present disclosure provides a preferred solvent for each reaction step.

The products of each reaction step described in the present disclosure can be purified by recrystallization under suitable conditions. The recrystallization solvents used are not particularly limited, and any solvent that can dissolve the crude product to a certain extent and precipitate crystals under certain conditions is included in the present disclosure. In addition, many similar modifications or equivalent substitutions in the art, or equivalents to the solvents, solvent combinations, and different proportions of solvent combinations described in the present disclosure, are considered to be within the scope of the present disclosure. Wherein, the solvent may be alcohol, ether, alkane, halogenated hydrocarbon, ester, ketone, aromatic hydrocarbon, acetonitrile, acetic acid, water, DMF, or a combination thereof; for example, water, acetic acid, methanol, ethanol, n-propanol, isopropanol, n-butanol, isobutanol, tert-butanol, petroleum ether, n-pentane, n-hexane, n-heptane, cyclohexane, DMF, tetrahydrofuran, ether, isopropyl ether, dioxane, methyl tert-butyl ether, dimethoxyethane, diethylene glycol dimethyl ether, triethylene glycol dimethyl ether, dichloromethane, 1,2-dichloroethane, chloroform, carbon tetrachloride, ethyl acetate, isopropyl acetate, acetone, butanone, benzene, toluene, xylene, or combinations thereof.

The water content in the solvent described in the present disclosure has no specific limitation; that is, the water content in the solvent does not affect the occurrence of the reaction described in the present disclosure. Any solvent containing a certain amount of water that can be used in the present disclosure to a certain extent is considered a solvent according to the present disclosure. For example, the water content in the solvent is approximately less than 0.05%, less than 0.1%, less than 0.2%, less than 0.5%, less than 5%, less than 10%, less than 25%, less than 30%, or 0%. In some embodiments, the water content of the solvent is within a certain range, which is more conducive to the reaction; for example, in the step of using ethanol as the reaction solvent, using anhydrous ethanol is more advantageous to the reaction. In some embodiments, the water content of the solvent exceeds a certain range, which may affect the progress of the reaction (for example, affecting the yield of the reaction), but does not affect the occurrence of the reaction.

The intermediate compounds involved in the present disclosure can be prepared by various synthesis methods well known to those skilled in the art, including the specific embodiments listed below, embodiments formed by their combination with other chemical synthesis methods, and equivalent substitutions well known to those skilled in the art. Preferred embodiments include, but are not limited to, embodiments of the present disclosure. However, they should be considered included in the scope of protection of the present disclosure.

The solvents used in the present disclosure can be obtained on the commercial market.

A hyphen ("-") not between two letters or symbols indicates the connecting site of the substituent. For example, C₁₋₆ alkyl carbonyl- refers to a C₁₋₆ alkyl that is connected to the rest of the molecule through a carbonyl. However, when the connecting site of the substituent is apparent to those skilled in the art, for example, in a halogen substituent, the "-" can be omitted.

When there is a dotted line " 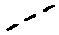 " across the radical valence bond, e.g., in 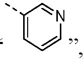 the wavy line indicates the point of attachment of this group to other parts of the molecule.

The term "hydrogen" used herein refers to the -H group.

The term "deuterium" used herein refers to the -D group.

The term "hydroxy" used herein refers to the -OH group.

The term "halo" or "halogen" used herein refers to fluorine (F), chlorine (Cl), bromine (Br), and iodine (I). The term "cyano" used herein refers to the -CN group.

Unless otherwise specified, Cₙ₋ₙ₊ₘ or Cₙ₋Cₙ₊ₘ includes any specific case of n to n+m carbons, such as C₁₋₁₂ including C₁, C₂, C₃, C₄, C₅, C₆, C₇, C₈, C₉, C₁₀, C₁₁, and C₁₂ and also comprising any range of n to n+m; for example, C₁₋₁₂ includes C₁₋₃, C₁₋₆, C₁₋₉, C₃₋₆, C₃₋₉, C₃₋₁₂, C₆₋₉, C₆₋₁₂, and C₉₋₁₂; similarly, n-membered to n+m-membered means that the number of atoms on a ring ranges from n to n+m. for example, 3-12 membered rings include 3-membered rings, 4-membered rings, 5-membered rings, 6-membered rings, 7-membered rings, 8-membered rings, 9-membered rings, 10-membered rings, 11-membered rings, and 12-membered rings, and also includes any range from n to n+m; for example, 3-12 membered rings include 3-6 membered rings, 3-9 membered rings, 5-6 membered rings, 5-7 membered rings, 6-7 membered rings, 6-8 membered rings, and 6-10 membered rings.

Unless otherwise specified, the number of atoms on a ring is usually defined as the number of elements of the ring, for example, "3-6 membered ring" refers to a "ring" of 3-6 atoms arranged around it.

Unless otherwise specified, the term "C₁₋₆ alkyl" is used to denote a linear or branched saturated hydrocarbon group consisting of 1 to 6 carbon atoms. The C₁₋₆ alkyl comprises C₁₋₅, C₁₋₄, C₁₋₃, C₁₋₂, C₂₋₆, C₂₋₄, C₆, C₅ alkyl, etc.; it may be monovalent such as CH₃), divalent (-CH₂-), or multivalent such as hypo 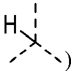 ). Examples of C₁₋₆ alkyl include, but are not limited to, CH₃, 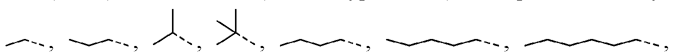 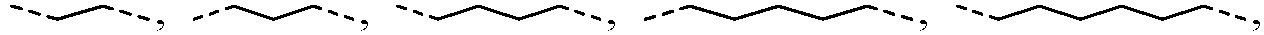 etc.

Unless otherwise specified, the term "C₁₋₃ alkyl" is used to denote a linear or branched saturated hydrocarbon consisting of 1 to 3 carbon atoms. The C₁₋₃ alkyl comprises C₁₋₂, C₂₋₃ alkyl, etc.; it may be monovalent (such as CH₃), divalent (-CH₂-), or multivalent (such as hypo 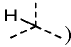 ). Examples of C₁₋₃ alkyl include, but are not limited to, CH₃, 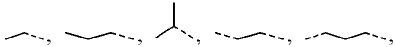 etc.

Unless otherwise specified, the term "C₁₋₆ alkoxy" refers to alkyl groups containing 1 to 6 carbon atoms that are connected to the rest of the molecule through an oxygen atom. The C₁₋₆ alkoxy includes C₁₋₄, C₁₋₃, C₁₋₂, C₂₋₆, C₂₋₄, C₆, C₅, C₄, and C₃ alkoxy. Examples of C₁₋₆ alkoxy include, but are not limited to, methoxy, ethoxy, propoxy (including n-propoxy and isopropoxy), butoxy (including n-butoxy, isobutyloxy, s-butoxy, and t-butoxy), pentoxy (including n-pentoxy, isopentoxy, and neopentoxy), hexoxy, etc.

Unless otherwise specified, the term "C₁₋₃ alkoxy" refers to alkyl groups containing 1 to 3 carbon atoms that are connected to the rest of the molecule through an oxygen atom. The C₁₋₃ alkoxy comprises C₁₋₂, C₂₋₃, C₃, C₂ alkoxy, etc. Examples of C₁₋₃ alkoxy include, but are not limited to, methoxy, ethoxy, propoxy (including n-propoxy and isopropoxy), etc.

Unless otherwise specified, the term "C₁₋₆ alkylamino" refers to alkyl groups containing 1 to 6 carbon atoms that are connected to the rest of the molecule through an amino. The C₁₋₆ alkylamino include C₁₋₄, C₁₋₃, C₁₋₂, C₂₋₆, C₂₋₄, C₆, C₅, C₄, C₃, C₂ alkylamino, etc. Examples of C₁₋₆ alkylamino acids include, but are not limited to, -NHCH₃,-N(CH₃)₂, -NHCH₂CH₃, -N(CH₃)CH₂CH₃, -N(CH₂CH₃)(CH₂CH₃), -NHCH₂CH₂CH₂CH₃, -NHCH₂(CH₃)₂,-NHCH₂CH₂CH₂CH₃, etc.

Unless otherwise specified, the term "C₁₋₃ alkylamino" refers to alkyl groups containing 1 to 3 carbon atoms that are connected to the rest of the molecule through an amino. The C₁₋₃ alkylamino includes C₁₋₂, C₃, C₂ alkylamino, etc. Examples of C₁₋₃ alkylamino acids include, but are not limited to, -NHCH₃, -N(CH₃)₂, -NHCH₂CH₃,-N(CH₃)CH₂CH₃, -NHCH₂CH₂CH₂CH₃, -NHCH₂(CH₃)₂, etc.

Unless otherwise specified, the term "C₁₋₆ alkylthio" refers to alkyl groups containing 1 to 6 carbon atoms that are connected to the rest of the molecule through sulfur atoms. The C₁₋₆ alkylthio includes C₁₋₄, C₁₋₃, C₁₋₂, C₂₋₆, C₂₋₄, C₆, C₅, C₄, C₃, and C₂ alkylthio. Examples of C₁₋₆ alkylthio include, but are not limited to, -SCH₃, -SCH₂CH₃,-SCH₂CH₂CH₃, -SCH₂(CH₃)₂, etc.

Unless otherwise specified, the term "C₁₋₃ alkylthio" refers to alkyl groups containing 1 to 3 carbon atoms that are connected to the rest of the molecule through sulfur atoms. The C₁₋₃ alkylthio comprises C₁₋₃, C₁₋₂, C₃ alkylthio, etc. Examples of C₁₋₃ alkylthio include, but are not limited to, -SCH₃, -SCH₂CH₃, -SCH₂CH₂CH₃, -SCH₂(CH₃)₂, etc.

Unless otherwise specified, "C₃₋₆ cycloalkyl" refers to a saturated cyclic hydrocarbon group consisting of 3 to 6 carbon atoms, which is a monocyclic and bicyclic system. The C₃₋₆ cycloalkyl includes C₃₋₅, C₄₋₅, and C₅₋₆ cycloalkyl, etc.; it may be monovalent, divalent, or multivalent. Examples of C₃₋₆ cycloalkyl include, but are not limited to, cyclopropyl, cyclobutyl, cyclopentyl, cyclohexyl, etc.

Unless otherwise specified, the term "3-6 membered heterocyclic alkyl", itself or in combination with other terms, respectively refers to a saturated cyclic consisting of 3 to 6 ring atoms, with 1, 2, 3, or 4 ring atoms being heteroatoms independently selected from O, S, and N, and the rest being carbon atoms, wherein the nitrogen atom is optionally quaternized, and the nitrogen and sulfur heteroatoms are optionally oxidized (i.e., NO and S(O)p, p being 1 or 2). It comprises a single ring and a double ring system, wherein the double ring system comprises a screw ring, a parallel ring, and a bridge ring. In addition, for this "3-6 membered heterocyclic alkyl," heteroatoms can occupy the connection position between the heterocyclic alkyl and the rest of the molecule. The 3-6 membered heterocyclic alkyl comprises 4-6 membered, 5-6 membered, 4-membered, 5-membered, and 6-membered heterocyclic alkyl. Examples of 3-6 membered heterocyclic alkyl include, but are not limited to, azacyclobutyl, oxocyclobutyl, thiocyclobutyl, pyrrolidinyl, pyrazolidine, imidazolidinyl, tetrahydrothienyl (including tetrahydrothienyl-2-yl, tetrahydrothienyl-3-yl, etc.), tetrahydrofuranyl (including tetrahydrofuran-2-yl, etc.), tetrahydropyranyl, piperidinyl (including 1-piperidinyl, 2-piperidinyl, 3-piperidinyl, etc.), piperazinyl (including 1-piperazinyl, 2-piperazinyl, etc.), morpholinyl (including 3-morpholinyl and 4-morpholinyl, etc.), dioxalkyl, dithialkyl, isoxazolyl, isothiazolyl, 1,2-oxazinyl, 1,2-thiazinyl, hexahydropyridazinyl, homopiperazinyl, or homopiperazinyl, etc.

Unless otherwise specified, the term "3-6 membered ring", itself or in combination with other terms, respectively refers to a saturated or unsaturated monocyclic group consisting of 3 to 6 ring atoms, which can include either a pure carbon ring or a ring containing heteroatoms. Wherein, 3-6 refers to the number of atoms forming the ring. Examples of "3-6 membered rings" include, but are not limited to, cyclopropyl, cyclobutyl, cyclopentyl, cyclohexyl, azacyclobutyl, oxyheterocyclobutyl, thioheterocyclobutyl, cyclopentanone, cyclohexanonyl, etc.

Unless otherwise specified, the terms "5-6 membered heteroaryl ring" and "5-6 membered heteroaryl" in the present disclosure can be used interchangeably. The term "5-6 membered heteroaryl" refers to a monocyclic group consisting of 5 to 6 ring atoms with a conjugated π electron system, of which 1, 2, 3, or 4 ring atoms are heteroatoms independently selected from O, S, and N, and the rest are carbon atoms. Wherein, the nitrogen atoms are optionally quaternized, and the nitrogen and sulfur heteroatoms are optionally oxidized (i.e., NO and S(O)ₚ, p being 1 or 2). 5-6 membered heteroaryls can be connected to the rest of the molecule through heteroatoms or carbon atoms. The 5-6 membered heteroaryl comprises 5-membered and 6-membered heteroaryls. Examples of the 5-6 membered heteroaryl include, but are not limited to, pyrrolyl (including N-pyrrolyl, 2-pyrrolyl, 3-pyrrolyl, etc.), pyrazolyl (including 2-pyrazolyl, 3-pyrazolyl, etc.), imidazolyl (including N-imidazolyl, 2-imidazolyl, 4-imidazolyl, 5-imidazolyl, etc.), oxazolyl (including 2-oxazolyl, 4-oxazolyl, 5-oxazolyl, etc.), triazolyl (1H-1,2,3-triazolyl, 2H-1,2,3-triazolyl, 1H-1,2,4-triazolyl, and 4H-1,2,4-triazolyl, etc.), tetrazolyl, isoxazolyl (3-isoxazolyl, 4-isoxazolyl, 5-isoxazolyl, etc.), thiazolyl (including 2-thiazolyl, 4-thiazolyl, 5-thiazolyl, etc.), furanyl (including 2-furanyl, 3-furanyl, etc.), thiophenyl (including 2-thienyl, 3-thienyl, etc.), pyridyl (including 2-pyridyl, 3-pyridyl, 4-pyridyl, etc.), pyrazinyl or pyrimidinyl (including 2-pyrimidinyl, 4-pyrimidinyl, etc.).

Accordingly, the term "heteroaromatic ring" as used herein refers to a heteroaryl ring as defined above.

As used herein, "aryl" and "aromatic" follow the Hückel's rule, where the number of π electrons equals 4n+2, and n is zero or any positive integer up to 6.

The term "carbonyl" used herein refers to the group -C(O)-, which can also be expressed as -CO-.

The term "amino" as used herein refers to the group -NH₂.

The term "optional" as used herein refers to the subsequent description of events that may or may not occur, and the description includes the circumstances in which the event occurs and the circumstances in which the event does not occur. For example, "optionally substituted alkyl" refers to unsubstituted alkyl and substituted alkyl, where alkyl is defined herein. Those skilled in the art should understand that for any group containing one or more substituents, the group does not include any spatially unrealistic, chemically incorrect, synthetically infeasible, and/or inherently unstable substitution modes.

As used herein, the term "substituted" or "substituted with" refers to the substitution of one or more hydrogen atoms in a given atom or group, such as by one or more substituents selected from a given substituent group, provided that the normal valence of the given atom is not exceeded. When the substituent is oxo (i.e. =O), the two hydrogen atoms in a single atom are replaced by oxygen. Such combinations are allowed only when combinations of substituents and/or variables result in chemically correct and stable compounds. A chemically correct and stable compound means that the compound is stable enough to be isolated from the reaction mixture and the chemical structure of the compound can be determined, and can subsequently be formulated into a formulation that has at least practical utility. For example, in the absence of a explicit list of substituents, the term "substituted" or "substitute" as used herein means that one or more hydrogen atoms on a given atom or group are independently substituted with one or more substituents, such as 1, 2, 3, or 4 substituents, the substituents being independently selected from: deuterium (D), halogen, -OH, mercapto, cyano, -CD₃, alkyl (preferably C₁₋₆ alkyl), alkoxy (preferably C₁₋₆ alkoxy), haloalkyl (preferably haloC₁₋₆alkyl), haloalkoxy (preferably haloC₁₋₆alkoxy), -C(O)NRₐR_{b}, and -N(Rₐ)C(O)R_{b}, and -C(O)OC₁₋₄alkyl (wherein Rₐ and R_{b} are each independently selected from hydrogen, C₁₋₄alkyl, haloC₁₋₄alkyl), carboxyl (COOH), cycloalkyl (preferably 3-8-membered cycloalkyl), heterocyclyl (preferably 3-8-membered heterocyclyl), aryl, heteroaryl, aryl-C₁₋₆alkyl-, heteroaryl-C₁₋₆alkyl-, OC₁₋₆ alkyl phenyl, -C₁₋₆ alkyl -OH (preferably -C₁₋₄ alkyl-OH), -C₁₋₆ alkyl -SH, -C₁₋₆ alkyl -O-C₁₋₂ alkyl, -C₁₋₆ alkyl -NH₂ (preferably -C₁₋₃ alkyl -NH₂), -N(C₁₋₆ alkyl)₂ (preferably -N(C₁₋₃ alkyl)₂), -NH(C₁₋₆ alkyl)(preferably -NH(C₁₋₃ alkyl)), -N(C₁₋₆ alkyl)(C₁₋₆ alkyl phenyl), -NH(C₁₋₆ alkyl phenyl), nitro, -C(O)OC₁₋₆ alkyl (preferably -C(O OC₁₋₃ alkyl), -NHC(O)(C₁₋₆ alkyl), -NHC(O)(phenyl)-N(C₁₋₆ alkyl)C(O)(C₁₋₆ alkyl), -N(C₁₋₆ alkyl)C(O)(phenyl), -C(O)C₁₋₆ alkyl, -C(O)heteroaryl (preferably -C(O)-5-7-membered heteroaryl), -C(O)C₁₋₆ alkylphenyl, -C(O)C₁₋₆ haloalkyl, -OC(O)C₁₋₆ alkyl (preferably -OC(O)C₁₋₃ alkyl), alkylsulfonyl (e.g., -S(O)₂-C₁₋₆ alkyl), alkylsulfinyl (-S(O)-C₁₋₆-alkyl), -S(O)₂-phenyl, -S(O)₂-C₁₋₆ haloalkyl,-S(O)₂NH₂, -S(O)₂NH(C₁₋₆ alkyl)-S(O)₂NH(phenyl), -NHS(O)₂(C₁₋₆ alkyl), -NHS(O)₂(phenyl), and -NHS(O)₂(C₁₋₆ haloalkyl), wherein the alkyl, cycloalkyl, phenyl, aryl, heterocyclyl, and heteroaryl are each optionally further substituted with one or more substituents selected from the following: halogen, -OH, -NH₂, cycloalkyl, 3-8-membered heterocyclic group, C₁₋₄ alkyl, C₁₋₄ haloalkyl -, -OC₁₋₄ alkyl, -C₁₋₄ alkyl -OH, -C₁₋₄ alkyl -O-C₁₋₄ alkyl,-OC₁₋₄ haloalkyl, cyano, nitro, -C(O)OH, -C(O)OC₁₋₆ alkyl, -CON(C₁₋₆ alkyl)₂, -CONH(C₁₋₆ alkyl), -CONH₂,-NHC(O)(C₁₋₆ alkyl), -NH(C₁₋₆ alkyl)C(O)(C₁₋₆ alkyl), -SO₂(C₁₋₆ alkyl), -SO₂(phenyl), -SO₂(C₁₋₆ haloalkyl), -SO₂NH₂, -SO₂NH(C₁₋₆ alkyl), -SO₂NH(phenyl), -NHSO₂(C₁₋₆ alkyl alkyl), -NHSO₂(phenyl), and -NHSO₂(C₁₋₆ haloalkyl). When an atom or group is substituted with multiple substituents, the substituents may be the same or different.

When any variable, such as R, appears more than once in the composition or structure of a compound, its definition in each case is independent. Thus, if, for example, one group is substituted with 0-2 of R, the groups may optionally be substituted with at most two of R, and there are independent options for R in each case. In addition, combinations of substituents and/or their variants are only permitted where such combinations will result in stable compounds.

The present disclosure employs the following acronyms:
SOCl₂ represents dichlorosulfoxide, Cs₂CO₃ represents cesium carbonate, NaHCO₃ represents sodium bicarbonate, K₂CO₃ represents potassium carbonate, K₃PO₄ represents potassium phosphate, DMF represents dimethylformamide, THF represents tetrahydrofuran, T3P represents 1-propylphosphoric anhydride, HATU represents 2-(7-azabenzotriazole)-N,N,N',N'-tetramethyluronium hexafluorophosphate, EDCI represents 1-(3-dimethylaminopropyl)-3-ethylcarbodiimide hydrochloride, HOBt represents 1-hydroxybenzotriazole, CDI represents N,N'-carbonyldiimidazole, DCM represents dichloromethane, DIPEA represents N,N-diisopropylethylamine, DCDMH represents dichlorodimethylheine, NCS represents N-chlorosuccinimide, MTBE represents methyl tert butyl ether, TCFH represents N,N,N',N'-tetramethylchloroformamidine hexafluorophosphate, and NMI represents N-methylimidazole.

The volume nV employed in the present disclosure generally refers to an n-fold volume as the solvent volume to weight ratio of the compound used in mL/g; for example, the ethanol volume 8V used in Table 3 refers to a volume of 8 times the volume of ethanol, i.e., the volume of ethanol (mL/) is 8 times the weight (g) of compound 1.

Compounds are named according to nomenclature principles routine in the art or by using ChemDraw^{®} Software nomenclature; for commercially available compounds, the vendor catalog names are used.

### DETAILED DESCRIPTION OF THE PREFERRED EMBODIMENTS

The application is described in detail by embodiments below, but this does not imply that there are any limitations which are detrimental to the application. The present application has been described in detail herein, wherein its specific embodiments are also disclosed, and it would be obvious to a person skilled in the art that various changes and improvements could be made with respect to the specific embodiments of the application without falling outside the spirit and scope of the present application.

No specific description of the starting materials used in the present disclosure is given, as all are commercially available.

### Embodiment 1

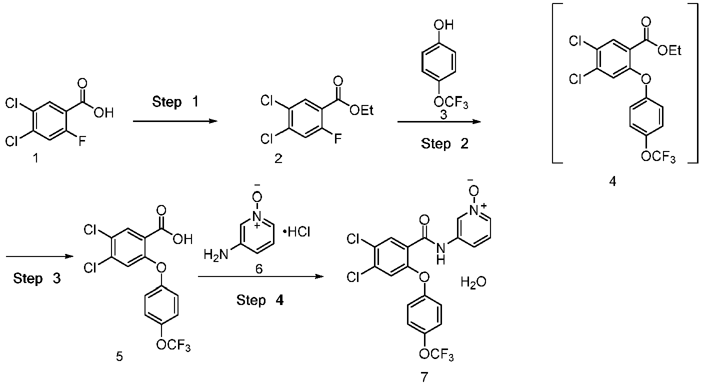

### Step 1

1) Reaction: adding anhydrous ethanol (38.4 kg, 6.45X) and compound 1 (5.95 kg, 1.0X) to reaction tank R1 under the protection of micro nitrogen, and activating stirring; adjusting the reaction tank R1 temperature to 0-10°C and, under the protection of micro nitrogen, controlling the reaction tank temperature to 0-20°C, slowly adding sulfoxide chloride (3.6 kg, 0.61X) dropwise into the reaction tank and, after dropwise addition, the temperature rising to 65-75°C, and stirring for 8-16 h; cooling to 40-50°C, sampling, and measuring by in-process control; qualification by in-process control (purity ratio of compound 1/(compound 1 + compound 2) ≤ 1.0%), then performing the next step; if qualification fails, continuing to stir the reaction until it qualifies.
2) Crystallization: reducing the temperature of the reaction tank 15-25°C and adding dropwise an aqueous solution of 10% potassium phosphate (60.0 kg, 10.1X) prepared in advance to adjust the pH to 6-8, then stirring for 3-6 h.
3) Centrifugation 1: centrifuging the material in the reaction tank using a centrifuge and washing the filter cake with process water (46.5 kg, 7.8X).
4) Slurrying: adding the wet product (7.0 kg, 1.2X) and the process water (61.2 kg, 10.3X) into the reaction tank R1 and stirring at 15-25°C for 3-6 h.
5) Centrifugation 2: centrifuging the material in the reaction tank using a centrifuge, and eluting the filter cake with process water (42.8 kg, 7.2X) to obtain a wet product, and after qualifying testing, using the wet product for the subsequent step.
6) Drying: transferring the wet product to a vacuum oven, controlling the oven internal temperature to 30-40°C and drying under reduced pressure for 24-30 h; sampling until water content is measured as qualifying (KF ≤ 0.3%); if it does not qualify, extending the drying time until the water content qualifies; obtaining from this batch a white solid of 6.1 kg at 90.4% yield.

1H NMR (400 MHz, DMSO-d6) δ 8.08-8.01 (m, 1H), 7.89 (d, J = 10.2 Hz, 1H), 4.32 (q, J = 7.1 Hz, 2H), 1.31 (t, J = 7.1 Hz, 3H).
HPLC conditions
Instrument: high-performance liquid chromatography (UV detector) Agilent 1260
Column: Waters XBridge C18/4.6 × 150 mm, 3.5 µm
Mobile phase: mobile phase a, 0.05% formic acid in water, mobile phase B, 0.05% formic acid in acetonitrile
Flow rate: 1.0 mL/min
Assay time: 28 min
Analytical temperature: 35°C
Detection wavelength: 222 nm/248 nm
Retention times: compound 1: 10.013 min; compound 2: 16.029 min

**Table 1**

| | Time (min) | A (%) | B (%) |
|---|---|---|---|
| Elution procedure | 0.0 | 90 | 10 |
| | 0.1 | 90 | 10 |
| | 6.0 | 55 | 45 |
| | 9.0 | 55 | 45 |
| | 16.0 | 10 | 90 |
| | 22.0 | 10 | 90 |
| | 22.1 | 90 | 10 |
| | 28.0 | 90 | 10 |

### Steps 2, 3

1) Reaction 1: adding compound 2 (6.0 kg, 1.00 X) and tetrahydrofuran (44.0 kg, 7.33 X) into the reaction tank under the protection of micro nitrogen and activating stirring; adding compound 3 (5.1 kg, 0.85 X), cesium carbonate (10.7 kg, 1.78 X) and stirring the reaction at 55-65°C for 16-24 hours, cooling to 15-25°C, and sampling and measuring by in-process control; after qualification by in-process control (purity ratio of compound 2/(compound 2 + compound 4 + compound 5) ≤ 4.0%), proceeding to the subsequent step; if in-process control does not result in qualification, then extending the reaction time until qualification by in-process control.
2) Reaction 2: adding the prepared aqueous solution of lithium hydroxide (35.8 kg, 6.0X) into reaction tank R1 under the protection of micro nitrogen gas, and controlling the temperature between 55-65°C, and stirring the reaction for 8-16 h; cooling to 15-25°C, and sampling and measuring by in-process control; after qualification by in-process control (purity ratio of compound 4/(compound 4 + compound 5) ≤ 1.0%), proceeding to the subsequent step; if in-process control does not result in qualification, extending the reaction time until the qualification by in-process control.
3) Acidification: controlling the temperature to 0-25°C under nitrogen protection, and slowly adding 35% hydrochloric acid (12 kg, 2.0X) dropwise into reaction tank R1.
4) Extraction: adding methyl tert butyl ether (21.5 kg, 3.6 X) to the reaction tank; adjusting the reaction tank temperature to 15-25°C, stirring for 0.5-2 hours, letting stand for 0.5-2 hours, allowing to separate, and transferring the aqueous phase to a steel plastic composite barrel.
5) Washing: adding the prepared 10% aqueous solution of sodium chloride (35.35 kg, 5.9X) into the reaction tank, adjusting the temperature of the reaction tank to 15-25°C, stirring for 0.5-2 h, letting stand for 0.5-2 h, allowing to separate, and transferring the aqueous phase to a steel plastic composite barrel.
6) Concentration: controlling the internal temperature to ≤ 40°C, concentrating the solution in the reaction tank to 12-18 L (2-3 X) under reduced pressure, adding anhydrous ethanol (25.0 kg, 4.2X); controlling the internal temperature to ≤ 40°C, concentrating the solution in the reaction tank to 12-18 L (2-3 X) under reduced pressure, and adding anhydrous ethanol (104.0 kg, 17.3 X); controlling the internal temperature to ≤ 40°C, concentrating the solution in the reaction tank to 12-18 L (2-3 X) under reduced pressure, and adding anhydrous ethanol (31.5 kg, 5.3 X) into the reaction tank R1.
7) Crystallization: controlling the reaction tank temperature to 50-60°C, adding process water (78.0 kg, 13.0 X) dropwise, and stirring for 1-3 h; cooling the reaction to 15-25°C, controlling the cooling time to be no less than 2 hours, and stirring was at 15-25°C for 1-3 hours.
8) Centrifugation: centrifuging the material in the reaction tank using a centrifuge, adding the prepared ethanol/process water = 1:1 (V/V) solution (26.1 kg, 4.3 X), washing the filter cake to obtain 8.60 kg of wet product, and after testing the wet product and it qualifying, proceeding to the subsequent step.
9) Drying: transferring the wet product to a vacuum oven, controlling the internal temperature to 40-50°C, and drying under reduced pressure for more than 18 hours until qualification (KF: 0.3% ≤ 0.3%, EtOH: 0.1% ≤ 0.2%); if not qualified, extending the drying time until qualification by in-process control; obtaining a white solid compound 7 (8.18 kg) at 85.4% yield.
   1H NMR (400 MHz, DMSO-d6) δ 8.05 (s, 1H), 7.50 (s, 1H), 7.42-7.32 (m, 2H), 7.12-7.02 (m, 2H).
   ESI⁺ [M+H]⁺: 366.9746, [M+H]⁺: 368.9719
   HPLC conditions
   Instrument: high-performance liquid chromatography (UV detector) Agilent 1260
   Column: Waters XBridge C18/4.6 × 150 mm, 3.5 µm
   Mobile phase: mobile phase a, 0.05% formic acid in water, mobile phase B, 0.05% formic acid in acetonitrile
   Flow rate: 1.0 mL/min
   Assay time: 28 min
   Analytical temperature: 35°C
   Detection wavelength: 222 nm/248 nm
   Retention times: compound 2: 16.029 min, compound 3: 10.712 min, compound 4: 18.792 min, and compound 5: 16.232 min.

### Step 4

1) Reaction: under nitrogen protection, adding DCM (56.5 kg, 6.93 X) and intermediate compound 5 (8.15 kg, 1.0 X) to the dry reaction tank, and activating stirring; adding the starting materials compound 6 (3.95 kg, 0.48 X) and HATU (10.2 kg, 1.25 X); controlling the temperature to 20-30°C, and adding DIPEA (8.5 kg, 1.05 X) dropwise into the reaction tank; after dropwise adding, warming to 25-35°C and stirring for 8-16 h; sampling and measuring by in-process control; after qualification by in-process control (purity ratio of compound 5/(compound 5 + compound 7) ≤ 1.0%), proceeding to the next step; if not qualified, extending the reaction time until qualification by in-process control.
2) Washing 1: cooling to 15-25°C and adding DCM (27.0 kg, 3.3X), prepared 5% aqueous K₂CO₃ (49.0 kg, 6.0X); controlling the internal temperature to 15-25°C and stirring for 0.5-2 hours; allowing to stand for 0.5-2 hours; allowing to separate, separating the aqueous phase, and retaining the organic phase.
3) Washing 2: a well configured 7% aqueous NaHCO₃ solution (81.0 kg, 9.9X) was added with controlled internal temperature of 15-25°C and stirred for 0.5-2 hours, and stand for 0.5-2 hours, separate the aqueous phase, and retain the organic phase. A well configured aqueous solution of 7% NaHCO₃ (83.0 kg, 10.2X) with controlled internal temperature of 15-25°C was added and stirred for 0.5-2 h, and stand for 0.5-2 h, then the solution was delaminated, the aqueous phase was separated, and the organic phase was retained.
4) Washing 3: adding process water to the organic phase (80.0 kg, 9.8X) and adding to the reaction tank R1; controlling the internal temperature to 15-25°C and stirring for 0.5-2 hours; letting stand for 0.5-2 hours; allowing to separate; evaporating the aqueous phase; retaining the organic phase; adding process water (80.8 kg, 9.9 X) into the reaction tank R1; controlling the internal temperature to 15-25°C and stirring for 0.5-2 h, and letting stand for 0.5-2 h, separating the aqueous phase, and retaining the organic phase.
5) Concentration: transferring the organic phase to the reaction tank, controlling the internal temperature to ≤ 35°C, and concentrating under reduced pressure to 16-24 L (2.0-3.0 X), and adding acetonitrile (97.0 kg, 11.9 X) to the reaction tank R1; controlling the internal temperature to ≤ 40°C, and continuing to concentrate the solvent under reduced pressure; concentrating the mixture to 73-81 L (9.0-10.0X) under reduced pressure, and adding acetonitrile (32.8 kg, 4.0X) to the reaction tank R1;with controlling the internal temperature to ≤ 40°C; continuing to concentrate the solvent to 73-81 L (9.0-10.0X) under reduced pressure; stopping the concentration.
6) Crystallization: warming the reaction to 50-60°C, and adding process water (135.2 kg, 16.6 X) dropwise into the reaction tank; cooling slowly to 40-50°C, stirring at 40-50°C for 2-4 hours, reducing the internal temperature to 0-10°C, and stirring for 6-8 hours.
7) Centrifugation: centrifuging the material in the reaction tank using a centrifuge, rinsing the filter cake with process water (17.5 kg, 2.1 X), sampling and testing for qualification (im-a ≤ 0.15%), then proceeding to the subsequent step; if not qualified, repeating crystallization.
8) Drying: transferring the filter cake to a vacuum oven, drying at 35-45°C under reduced pressure for 18-24 hours until the water content (KF ≤ 5.0%) qualifies; obtaining compound 7 (9.68 kg) at 91.4% yield; testing that the water content of compound 7 shows a compound to water molar ratio of 1:1 and TGA shows a weight loss of 3.5% from 50-150 degrees; converting to a molar ratio approaching 1:1 by the molecular weight of water.
   ESI⁺ [M+H]⁺: 459.0019, [M+H]⁺: 461.0090
   1H NMR (400 MHz, DMSO-d6) δ 10.86 (s, 1H), 8.61 (s, 1H), 8.03 (s, 1H), 8.00 (ddd, J = 6.3, 1.8, 1.0 Hz, 1H), 7.48-7.43 (m, 2H), 7.41-7.34 (m, 3H), 7.20-7.15 (m, 2H).
   HPLC conditions
   Instrument: high-performance liquid chromatography (UV detector) Agilent 1260
   Column: Waters XBridge C18/4.6 × 150 mm, 3.5 µm
   Mobile phase: mobile phase a, 0.05% formic acid in water, mobile phase B, 0.05% formic acid in acetonitrile
   Flow rate: 1.0 mL/min
   Assay time: 28 min
   Analytical temperature: 35°C
   Detection wavelength: 222 nm/248 nm
   Retention times: compound 5: 16.232 min; compound 6: 1.729 min; and compound 7: 13.976 min.

### Embodiment 2

Step 1: adding anhydrous ethanol (464 ml, 8.0 V/W) and compound 1 (58.0 g, 278 mmol) to the reaction tank at room temperature; stirring the solution; cooling to 30-40°C, SOCl₂ (39.6 g, 333 mmol) was added dropwise; after dropwise addition, heating to 55-60°C for 4-8 hours, and sampling and performing in-process control until the reaction qualifies; adjusting pH to 6-8 by dropwise addition of 10% potassium phosphate solution; precipitating the product, filtering, and washing the filter cake to obtain a product; drying at 30-40°C to obtain compound 2.

Steps 2, 3: adding THF (130 ml, 8.0 V/W) to a three-necked flask at room temperature and stirring; adding compound 2 (56.2 g, 237 mmol), compound 3 (48.5 g, 272 mmol), and Cs₂CO₃ (100.4 g, 308 mmol) and heating to 55-60°C for 20-24 h; sampling with in-process control; adding water (280 ml) and lithium hydroxide monohydrate (24.9 g, 593 mmol) at 55-60°C for 2-4 h; sampling and measuring; testing with in-process control; after qualification with in-process control, cooling to 20-30°C; adding concentrated hydrochloric acid (101 ml, 1.8 vol) dropwise, then adding MTBE (130 ml) and stirring for 10-15 min; allowing to separate; adding 10% sodium chloride solution (280 ml, 5 vol) to the organic phase and washing; concentrating the organic phase to 2-3 volumes under reduced pressure; adding anhydrous ethanol (280 ml, 5 V) and continuing concentration to 2-3 volumes; adding anhydrous ethanol to approximately 10 volumes; adding water (504 ml, 9 V) dropwise at 20-30°C; precipitating a solid; filtering to obtain a wet product; drying at 40-50°C to obtain compound 5.

Synthesis of step 4: adding DCM (200 ml), compound 5 (40.0 g, 109 mmol) to a three-necked flask at room temperature, and beginning stirring; adding HATU (53.8 g, 144 mmol) and compound 6 (19.1 g, 131 mmol); adding DIPEA (42.2 g, 327mmol) dropwise at 30-35°C; after dropwise addition is complete, reacting at 30-35°C for 16 hours; sampling and in-process control, and after qualification, cooling the system to 25-35°C; adding DCM (100 ml), adding 5% potassium carbonate aqueous solution (240 ml, 6 V) and washing; allowing to stand and separate, adding 7% sodium bicarbonate aqueous solution (400 ml, 10 V) to the organic phase and washing twice; adding water (400 ml, 10 V) and washing twice; concentrating the organic phase under reduced pressure until 3.0-3.5 V remains; adding acetonitrile (480 ml, 12 V) and continuing to concentrate the replacement solvent twice; concentrating until 10 V remains; warming the system to 50-60°C, adding water (560 ml, 14 V) dropwise; then, keeping warm at 50-60°C for 1 h; cooling to 0-10°C and stirring for 2 h; filtering to obtain a wet product; drying under reduced pressure at 45-50°C to obtain compound 7.

### Embodiment 3

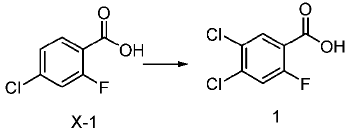

Adding concentrated sulfuric acid (5 ml, 5 V) to the reaction flask, then adding compound X-1 (1 g, 1.0 eq) at 20-25°C; adding DCDMH (0.55 eq) in batches; warming to 50-55°C, and reacting overnight; in-process control until qualification, then cooling the reaction liquid to 20-25°C; adding the reaction liquid dropwise into a 20V ice water bath; after dropwise addition, restoring to 20-25°C and stirring for 2 hours; filtering; adding water and washing the filter cake to obtain a wet product, and purifying same; drying at 50-60°C to obtain compound 1 at a yield of 50-60%.

### Embodiment 4

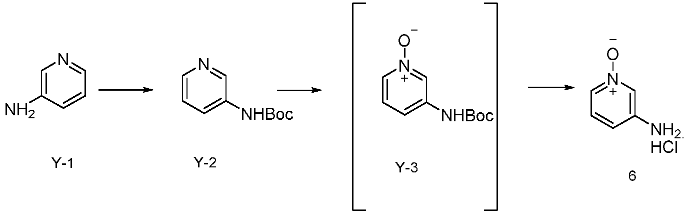

**Synthesis of compound Y-2:** Adding IPA (30.0 ml, 3 V) and water (30.0 ml, 3 V), Y-1 (10.0 g, 106 mmol) to a reaction flask at room temperature, and stirring the solution; cooling to 0-5°C, and adding Boc₂O (27.8 g, 127 mmol)/IPA (3.0 V) dropwise; after dropwise addition is complete, returning the reaction to room temperature for 2-3 hours; sampling with in-process control for qualification; at 40-50°C, concentrating the reaction under reduced pressure and adding MTBE (100 ml) to dissolve; washing with water (100.0 ml, 10 V) and allowing to separate; concentrating the organic phase to dry to obtain product Y-2.

**Synthesis of compound Y-3:** Adding Y-2 (10 g, 52 mmol) and DCM (100 ml, 10 V) to a reaction flask at room temperature, and stirring; cooling to 0-10°C, adding solid m-CPBA in batches, returning the temperature to 20-25°C and reacting overnight; sampling with in-process control for qualification; adding 10% Na₂SO₃ (20 ml, 2 V) to the reaction flask and washing once; adding another 10% Na₂SO₃ (20 ml, 2 V) to the reaction flask and washing once; allowing to separate; adding ethyl acetate (50 ml, 5 V) to the aqueous phase and extracting; concentrating the organic phase to dry; adding ethyl acetate (100 ml, 10 V) and combining with ethyl acetate phases twice; passing over a pad of silica gel to obtain a filtrate; concentrating the filtrate until dry, and adding 1.4-dioxane (50 ml, 5 V) to dissolve to obtain the Y-3 solution.

**Synthesis of compound 6:** At room temperature, adding Y-3 solution (52 mmol) to the reaction flask, controlling the temperature to 10-20°C, and adding HCl/1,4-dioxane solution (520 mmol, 10.0 eq.) dropwise; after dropwise addition, returning to room temperature for reaction for 3-4 hours; sampling with in-process control for qualification of the reaction; during the reaction process, separating solids out and filtering to obtain a filter cake; adding the filter cake to the reaction flask, slurrying with acetonitrile (50ml, 5V) at room temperature for 1-2 hours, and filtering to obtain a wet product; drying at 45-50°C to obtain a compound 6.

### Embodiment 5

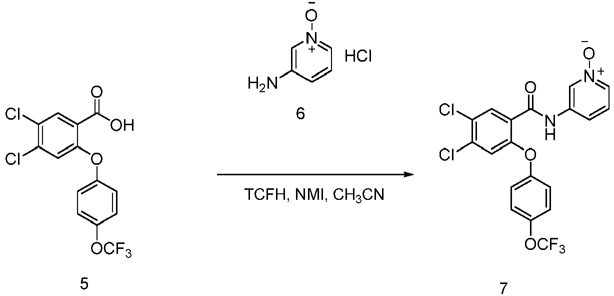

Under nitrogen protection, adding CH₃CN (62.2 kg) and compound 5 (13.5 kg) to a dry reactor R1 and activating stirring; adding compound 6 (6.5 kg) and TCFH (15.5 kg); controlling to 0-10°C and adding NMI (10.5 kg) dropwise into the reactor; after dropwise addition, warming to 25-35 °C and stirring for 2-4 hours; sampling with in-process control and, after qualification by in-process control, proceeding to the subsequent step; controlling to 25-35 °C, adding a prepared 3% K₃PO₄ aqueous solution (138 kg), and controlling the internal temperature to 25-35 °C, and stirring for 2-4 hours; transferring the material in the reactor to a centrifuge for centrifugation; rinsing the filter cake with process water (37.1 kg); adding the above wet product and process water (140 kg) to the reactor R1 and stirring at 35-45° C for 2-4 hours; transferring the material in the reactor to a centrifuge for centrifugation; adding process water (211.7 kg) to rinse the filter cake; adding the above wet product and process water (140 kg) into the reactor R1, and stirring at 35-45 °C for 2-4 hours; transferring the material in the reactor to a centrifuge for centrifugation, and adding process water (185.4 kg) to rinse the filter cake; transferring the filter cake to a vacuum oven and drying under reduced pressure at 35-45 °C for 18-24 hours until the water content (KF≤5.0%) qualifies; obtaining compound 7 (15.7 kg) at 85% yield.

### Comparative Example 1

Keeping the other conditions consistent with those in step 1 of embodiment 2, and changing the amount of SOCl₂ in step 1 only, the results are shown in Table 2 below.

**Table 2**

| Equivalent (eq.) | Reaction in-process control | | Compound 2 |
|---|---|---|---|
| | Purity ratio of compound 1/(compound 1 + compound 2) | Mass ratio of compound 2/(compound 1 + compound 2) | Purity |
| 1.5 | 0.15% | 99.5% | 99.2% |
| 1.2 | 0.34% | 99.1% | 99.3% |

### Comparative Example 2

Keeping the other conditions consistent with those in step 1 of embodiment 2, and changing the amount of ethanol by volume in step 1 only, the results are shown in Table 3 below.

**Table 3**

| Volume of ethanol | Reaction in-process control | | Compound 2 | |
|---|---|---|---|---|
| | Purity ratio of compound 1/(compound 1 + compound 2) | Mass ratio of compound 2/(compound 1 + compound 2) | Purity | Yield |
| 10V | 0.41% | 99.0% | 99.1% | 94.6% |
| 8 V | 0.34% | 99.1% | 99.3% | 92.8% |

### Comparative Example 3

Keeping the other conditions consistent with those in step 1 of embodiment 2, and changing the post-treatment operation in step 1 only, the results are shown in Table 4 below.

**Table 4**

| Post-treatment method | Compound 2 | |
|---|---|---|
| | Purity | Yield |
| First concentrating the ethanol, then extracting with ethyl acetate and water, then alkaline washing, then concentrating until dry | 99.1% | 94.6% |
| Cooling the system to 20-30°C, adding dropwise aqueous sodium bicarbonate solution, precipitating the product, suction filtering, water eluting, then drying | 99.3% | 92.8% |

### Comparative Example 4

Keeping the other conditions consistent with those in step 2 of embodiment 2, and changing the choice of alkali in step 2 only, the results are shown in Table 5 below.

**Table 5**

| Alkali | Reaction time | Reaction in-process control | | Experimental result |
|---|---|---|---|---|
| | | Purity ratio of compound 2/(compound 2 + compound 4 + compound 5) | Purity ratio of compound 4/(compound 2 + compound 4 + compound 5) | |
| Cs₂CO₃ | 23h | 7.7% | 86.8% | Higher conversion rate |
| NaHCO₃ | 17 h | 40.0% | Reaction system becomes heterogenous | Purity of reaction system becomes obviously heterogenous after overnight incubation |
| K₂CO₃ | 17 h | 15.3% | 84.7% | Conversion rate is insufficient |
| K₃PO₄ | 20 h | 1.8% | 71.5% | More hydrolysis of starting materials |

### Comparative Example 5

Keeping the other conditions consistent with those in step 2 of embodiment 2, and changing the choice of solvent in step 2 only, the results are shown in Table 6 below.

**Table 6**

| Solvent | Reaction in-process control | Compound 4 | | |
|---|---|---|---|---|
| | Purity ratio of compound 2/(compound 2 + compound 4 + compound 5) | Purity | Yield | Post-treatment results |
| DMF | 7.7% | 86.8% | -100% | Yellow oil substance |
| THF | 1.5% | 90.8% | NA | Two-step continuous reaction |

### Comparative Example 6

Keeping the other conditions consistent with those in step 2 of embodiment 2, and changing the choice of cesium carbonate in step 2 only, the results are shown in Table 7 below.

**Table 7**

| Cs₂CO₃ | | Reaction in-process control | Compound 5 | |
|---|---|---|---|---|
| | Reaction time | Purity ratio of compound 2/(compound 2 + compound 4 + compound 5) | Purity | Yield |
| 1.5 eq. | 20 h | NA | 98.2% | 58.5% |
| 1.4 eq. | 16 h | 2.0% | 98.8% | 68.1% |
| 1.3 eq. | 20 h | 0.6% | 99.2% | 74.1% |

### Comparative Example 7

Keeping the other conditions consistent with those in step 2 of embodiment, and changing the amount of compound 3 in step 2 only, the results are shown in Table 7 below.

**Table 8**

| Compound 3 | Reaction time | Reaction in-process control | Compound 5 | |
|---|---|---|---|---|
| | | Purity ratio of compound 2/(compound 2 + compound 4 + compound 5) | Purity | Yield |
| 1.25 eq. | 16 h | 2.0% | 98.8% | 68.1% |
| 1.10 eq. | 25 h | 1.5% | 99.7% | 64.0% |
| 1.15 eq. | 20 h | 0.6% | 99.2% | 74.1% |

### Comparative Example 8

Keeping the other conditions consistent with those in step 4 of embodiment 2, and changing the type of condensing agent in step 4 only, the results are shown in Table 9 below.

**Table 9**

| Condensing agent | Reaction in-process control | | Reaction time |
|---|---|---|---|
| | Purity ratio of compound 5/(compound 5 + compound 7) | Compound 7 | |
| T3P | 100% | Undetected | 14 h |
| HATU | 0.00% | 98.75% | 16 h |
| Oxalyl chloride | 100% | Undetected | 22 h |
| EDCI, HOBt | 16.50% | 63.56% | 23 h |
| CDI | 98.39% | 1.59% | 23 h |
| TCFH, NMI | 0.0% | 97.65% | 4h |

### Comparative Example 9

Keeping the other conditions consistent with those in step 4 of embodiment 2, and changing the type of solvent in step 4 only, the results are shown in Table 10 below.

**Table 10**

| Solvent | Reaction in-process control | | Reaction time |
|---|---|---|---|
| | Purity ratio of compound 5/(compound 5 + compound 7) | Compound 7 | |
| DMF | 0.00% | 98.75% | 16 h |
| Acetonitrile | 2.67% | 80.01% | 22 h |
| DCM | 0.90% | 95.27% | 5h |

### Comparative Example 10

Keeping the other conditions consistent with those in step 4 of embodiment 2, and changing the amount of HATU in step 4 only, the results are shown in Table 11 below.

**Table 11**

| HATU | Reaction in-process control | | Reaction time |
|---|---|---|---|
| | Purity ratio of compound 5/(compound 5 + compound 7) | Compound 7 | |
| 1.5 eq. | 0.90% | 95.27% | 5h |
| 1.2 eq. | 0.96% | 96.94% | 16 h |

### Comparative Example 11

Keeping the other conditions consistent with those in step 4 of embodiment 2, and changing the amount of DIPEA in step 4 only, and the results are shown in Table 12 below.

**Table 12**

| DIPEA | Reaction in-process control | | Reaction time |
|---|---|---|---|
| | Purity ratio of compound 5/(compound 5 + compound 7) | Compound 7 | |
| 3.0 eq. | 0.96% | 96.94% | 16 h |
| 2.5 eq. | 7.92% | 90.10% | 16 h |

### Comparative Example 12

Keeping the other conditions consistent with those in step 4 of embodiment 2, and changing the post-treatment conditions in step 4 only, the results are shown in Table 13 below.

**Table 13**

| Washed with 20% citric acid aqueous solution? | Compound 7 | | Remarks |
|---|---|---|---|
| | Purity | Yield | |
| Yes | > 99.5% | 76.0% | Risk of precipitated product |
| No | > 99.5% | 80.8% | No risk of precipitated product |

### Comparative Example 13

Screening the reaction conditions of embodiment 3, the results are shown in Table 14 below.

**Table 14**

| **Material** | | | **Reaction conditions** | | **In-process control conditions** |
|---|---|---|---|---|---|
| **Starting material** | **Reagent** | **Solvent** | **Temperature** | **Time** | |
| **X-1** (1.0 g, 1.0 eq) | NCS (1.1 eq) | H₂SO₄ (5 v) | 25°C | 2h | X-1: 97% |
| | | | | | Compound 1: 3% |
| | | | 45°C | 3 h | X-1: 70% |
| | | | | | Compound 1: 30% |
| | | | 60°C | 14 h | X-1: 21% |
| | | | | | Compound 1: 79% |
| **X-1** (1.0 g, 1.0 eq) | NCS (1.1 eq) | DCM (5 v) | 25°C | 2h | No reaction |
| | | | 45°C | 3 h | No reaction |
| **X-1** (1.0 g, 1.0 eq) | NCS (1.1 eq) | DMF (5 v) | 25°C | 2h | No reaction |
| | | | 45°C | 3 h | No reaction |
| **X-1** (1.0 g, 1.0 eq) | NCS (1.1 eq) | MeCN (5 v) | 25°C | 2h | No reaction |
| | | | 45°C | 3 h | No reaction |
| **X-1** (1.0 g, 1.0 eq) | NCS (1.2 eq) | H₂SO₄ (5 v) | 60°C | 14 h | X-1: 22% |
| | | | | | Compound 1: 78% |
| **X-1** (1.0 g, 1.0 eq) | NCS (1.4 eq) | H₂SO₄ (5 v) | 60°C | 14 h | X-1: 16% |
| | | | | | Compound 1: 84% |
| **X-1** (1.0 g, 1.0 eq) | NCS (1.6 eq) | H₂SO₄ (5 v) | 60°C | 14 h | X-1: 15.5% |
| | | | | | Compound 1: 84.5% |
| **X-1** (1.0 g, 1.0 eq) | NCS (1.2 eq) | AcOH (5 v) | 60°C | 14 h | No reaction |
| **X-1** (1.0 g, 1.0 eq) | DCDMH (0.55 eq) | H₂SO₄ (5 v) | 45°C | 9h | X-1: 11.8% |
| | | | | | Compound 1: 86.7% |
| | | | 60°C | 12 h | X-1: 2.6% |
| | | | | | Compound 1: 93.8% |
| **X-1** (1.0 g, 1.0 eq) | DCDMH (0.5 eq) | H₂SO₄ (5 v) | 45°C | 15 h | X-1: 7.6% |
| | | | | | Compound 1: 90.6% |
| **X-1** (1.0 g, 1.0 eq) | DCDMH (0.6 eq) | H₂SO₄ (5 v) | 45°C | 15 h | X-1: 4.8% |
| | | | | | Compound 1: 92.6% |
| **X-1** (1.0 g, 1.0 eq) | DCDMH (0.7 eq) | H₂SO₄ (5 v) | 45°C | 15 h | X-1: 4.8% |
| | | | | | Compound 1: 92.4% |
| **X-1** (1.0 g, 1.0 eq) | DCDMH (0.55 eq) | H₂SO₄ (5 v) | 25°C | 15 h | X-1: 39.3% |
| | | | | | Compound 1: 60.5% |
| | | | 35°C | 20 h | X-1: 8.8% |
| | | | | | Compound 1: 89.9% |

In the descriptions of the present specification, a description referring to the terms "one embodiment", "some embodiments", "example", "specific example", or "some examples", etc. means that a specific feature, structure, material, or characteristic described in combination with that embodiment or example is included in at least one embodiment or example of the disclosure. In the present specification, schematic representations of the above terms do not necessarily have to concern the same embodiments or examples. Moreover, the specific features, structures, materials or characteristics described may be combined in a suitable manner in any one or more embodiments or examples. Furthermore, without contradiction, a person skilled in the art may combine different embodiments or examples described in the present specification with the features of different embodiments or examples.

The implementations of the technical solutions of the present disclosure have been described above. It should be understood that the scope of protection of the present application should not be limited to the implementations described above. Any modifications, equivalent substitutions, improvements or the like made by those skilled in the art without departing from the spirit and principle of the present disclosure shall fall within the scope of protection of the present application.

## Claims

1. A method for preparing a compound represented by formula (I) or a pharmaceutically acceptable salt or hydrate thereof, wherein it comprises the compound represented by formula (I-5) or a pharmaceutically acceptable salt thereof coming into contact with the compound represented by formula (I-6) or a pharmaceutically acceptable salt thereof to obtain a compound represented by formula (I) or a pharmaceutically acceptable salt thereof or hydrate thereof, 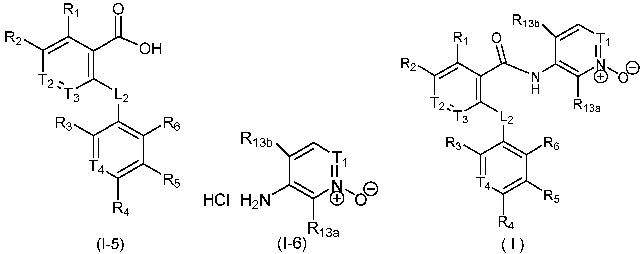
T₁ is selected from N or C(R₇);
T₂ is selected from N or C(R₈);
T₃ is selected from N or C(R₉);
T₄ is selected from N or C(R₁₀);
R₁, R₂, R₈, and R₉ are independently selected from H, halogen, OH, NH₂, CN, SF₅, C₁₋₆ alkyl, C₁₋₆ alkoxy, C₁₋₆ alkylamino, vinyl-C₁₋₆ alkyl-, C₃₋₆ cycloalkyl, 3-6 membered heterocycloalkyl, C₃₋₆ cycloalkyl-C₁₋₆ alkyl-, 3-6 membered heterocycloalkyl-C₁₋₆ alkyl-, 3-6 membered heterocycloalkyl-C₁₋₆ alkyl-, phenyl-C₁₋₃ alkyl-, C₃₋₆ cycloalkyl-C₁₋₃ alkyl-, 3-6 membered heterocycloalkyl-C₁₋₃ alkyl-, phenyl-C₁₋₃ alkyl-phenyl-C₁₋₃ alkyl-NH-, 5-6 membered heteroaryl-C₁₋₃ alkyl-, 5-6 membered heteroaryl-C₁₋₃ alkyl-O-, and 5-6 membered heteroaryl-C₁₋₃ alkyl-NH-, the C₁₋₆ alkyl, C₁₋₆ alkoxy, C₁₋₆ alkylamino, vinyl-C₁₋₆ alkyl-, C₃₋₆ cycloalkyl, 3-6 membered heterocycloalkyl, C₃₋₆ cycloalkyl-C₁₋₆ alkyl-, 3-6 membered heterocycloalkyl-C₁₋₆ alkyl-, 3-6 membered heterocycloalkyl-C₁₋₆ alkyl-, phenyl-C₁₋₃ alkyl-, C₃₋₆ cycloalkyl-C₁₋₃ alkyl-O-, 3-6 membered heterocycloalkyl-C₁₋₃ alkyl-O-, phenyl-C₁₋₃ alkyl-O-, phenyl-C₁₋₃ alkyl-NH-, 5-6 membered heteroaryl-C₁₋₃ alkyl-O-, or 5-6 membered heteroaryl-C₁₋₃ alkyl-NH-, optionally substituted with 1, 2, or 3 of R;
R₃, R₄, R₅, R₆, and R₁₀ are independently selected from H, halogen, OH, NH₂, SF₅, CN, C₁₋₆ alkyl, C₁₋₆ alkylamino, C₁₋₆ alkoxy, C₃₋₆ cycloalkyl, -O-C₃₋₆ cycloalkyl, 3-6 membered heterocycloalkyl, C₃₋₆ cycloalkyl-C₁₋₆ alkyl-, and 3-6 membered heterocycloalkyl-C₁₋₆ alkyl-, respectively, the C₁₋₆ alkyl, C₁₋₆ alkylamino, C₁₋₆ alkoxy, C₃₋₆ cycloalkyl, -O-C₃₋₆ cycloalkyl, 3-6 membered heterocycloalkyl C₃₋₆ cycloalkyl-C₁₋₆ alkyl- or 3-6 membered heterocycloalkyl-C₁₋₆ alkyl-, optionally substituted with 1, 2, or 3 of R;
R₇ is selected from H, F, Cl, Br, I, C₁₋₆ alkyl, C₁₋₆ alkoxy, and C₁₋₆ alkylamino, the C₁₋₆ alkyl, C₁₋₆ alkoxy, or C₁₋₆ alkylamino, optionally substituted with 1, 2, or 3 of R;
L₂ is selected from O, S, NH, and CH₂, wherein CH₂ is optionally substituted with 1 or 2 of R and NH is optionally substituted with R;
R₁₃ₐ and R_{13b} are independently selected from H, halogen, and C₁₋₆ alkyl, wherein C₁₋₆ alkyl is optionally substituted with 1, 2, or 3 of R;
R is independently selected from H, D, halogen, OH, NH₂, CN, 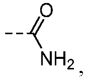
C₁₋₆ alkyl, C₁₋₆ alkoxy, C₁₋₆ alkylthio, and C₁₋₆ alkylamino, the C₁₋₆ alkyl, C₁₋₆ alkoxy, C₁₋₆ alkylthio, or C₁₋₆ alkylamino, optionally substituted with 1, 2, or 3 of R';
R' is selected from F, Cl, Br, I, OH, NH, and CH₂;
the above 3-6 membered heterocycloalkyl or 5-6 membered heteroaryl comprises 1, 2, or 3 heteroatoms or heteroatomic groups independently selected from-O-, -NH-, -S-, -C (=O)-, -C (=O)O-, -S(=O)-, -S(=O)₂-, and N.

2. The method according to claim 1, wherein, the compound represented by formula (I) is selected from 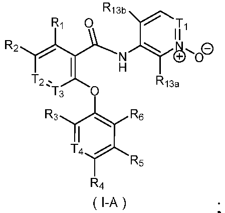
optionally, the compound represented by formula (I) is selected from 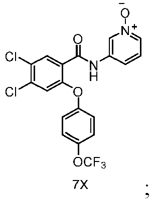
optionally, the compound represented by formula (I) is selected from 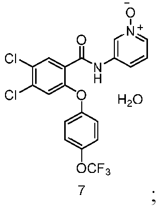
optionally, the compound represented by formula (I-5) is selected from 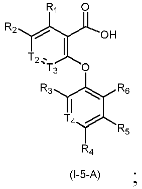
optionally, the compound represented by formula (I-5) is selected from 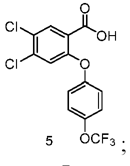 optionally, the compound represented by formula (I-6) is selected from 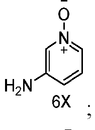
optionally, the compound represented by formula (I-6) is selected from

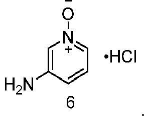

3. The method according to claim 1, wherein, the contact is carried out under the conditions of a condensation agent;
optionally, the condensation agent is selected from 2-(7-azabenzotriazole)-N,N',N'-tetramethyluronium hexafluorophosphate, 1-(3-dimethylaminopropyl)-3-ethylcarbodiimide hydrochloride/1-hydroxybenzotriazole, N,NN'-carbonyldiimidazole, N,N,N',N'-tetramethylchloroformamidine hexafluorophosphate, or N-methylimidazole;
optionally, the condensation agent is 2-(7-azabenzotriazole)-N,N,N',N'-tetramethyluronium hexafluorophosphate;
optionally, the condensation agent is N,N,N',N'-tetramethylchloroformamidine hexafluorophosphate and N-methylimidazole;
optionally, the molar ratio of the compound represented by formula (I-5) to the 2-(7-azabenzotriazole)-N,N,N',N'-tetramethyluronium hexafluorophosphate is 1:(1.0-2.0), and optionally 1:(1.2-1.5);
optionally, the molar ratio of the compound represented by formula (I-5) to the N,N,N',N'-tetramethylchloroformamidine hexafluorophosphate is 1:(1.0-2.0), and optionally 1:(1.1-1.2);
optionally, the molar ratio of N-Methylimidazole to the N,N,N',N'-Tetramethylchloroformamidine hexafluorophosphate is 1:(1.2-2.0), and optionally 1:(1.4-1.6);
optionally, the contact is carried out under alkaline conditions;
optionally, the alkaline condition is provided by N,N-diisopropylethylamine;
optionally, the molar ratio of the compound represented by formula (I-5) to the N,N-diisopropylethylamine is 1:(1.0-5.0), and optionally 1:(2.5-3.0);
optionally, the contact is carried out under conditions where the solvent is N,N-dimethylformamide, acetonitrile, or dichloromethane;
optionally, the contact is carried out under the condition that the solvent is N,N-dimethylformamide.

4. The method according to claim 2, wherein, the compound represented by formula (I-5-A) is obtained by a substitution reaction between the compound represented by formula (I-2) and the compound represented by formula (I-3), 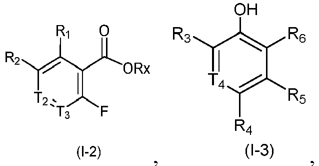
wherein Rx is selected from C₁₋₆ alkyl;
optionally, the compound represented by formula (I-2) or its optical isomer is selected from 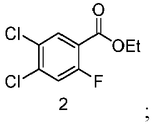
optionally, the compound represented by formula (I-3) or its optical isomer is selected from 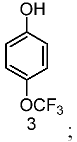 optionally, the substitution reaction is carried out in the presence of an alkali;
optionally, the alkali is selected from Cs₂CO₃, K₂CO₃, and K₃PO₄;
optionally, the alkali is Cs₂CO₃;
optionally, the molar ratio of the compound represented by formula (I-2) to the Cs₂CO₃ is 1:(1.3-1.5);
optionally, the substitution reaction is carried out in a solvent of N,N-dimethylformamide or tetrahydrofuran;
optionally, the molar ratio of the compound represented by formula (I-2) to the compound represented by formula (I-3) is 1:(1.10-1.25), and optionally 1:1.15.

5. The method according to claim 4, wherein, the compound represented by formula (I-2) is obtained by esterification of the compound represented by formula (I-1), 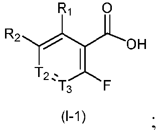
optionally, the compound represented by formula (I-1) or its optical isomer is selected from 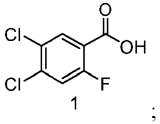
optionally, the esterification reaction is carried out in the presence of SOCl₂;
optionally, the molar ratio of the compound represented by formula (I-1) to the SOCl₂ is 1:(1.0-2.0), and optionally 1:(1.2-1.5);
optionally, the esterification reaction is carried out under the condition that the solvent is ethanol;
optionally, the ratio of the compound represented by formula (I-1) to the ethanol is 1:(1672-2090);
optionally, the esterification reaction further comprises a post-treatment;
optionally, the post-treatment is performed by adding an aqueous solution of sodium bicarbonate.

6. An intermediate compound or pharmaceutically acceptable salt thereof, wherein, its structure is represented by compound 5,

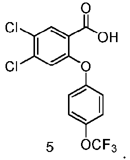

7. A use of the compound represented by compound 5 or a pharmaceutically acceptable salt thereof in the preparation of the compound represented by compound 7X or its optical isomers.

8. A method for preparing compound 1, wherein, it comprises performing a chlorination reaction on the compound represented by formula (X-1) to obtain compound 1, the chlorination reaction being carried out in one or more solvent of concentrated sulfuric acid, methanesulfonic acid, trifluoroacetic acid, phosphoric acid, or ethanesulfonic acid,

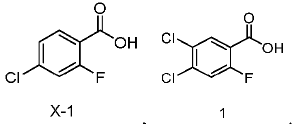

9. The method according to claim 8, wherein the chlorination reaction is carried out under the conditions of a chlorination reagent;
optionally, the chlorination reagent is selected from 1,3-dichloro-5,5-dimethylhydantoin and N-chlorosuccinimide;
optionally, the molar ratio of the compound represented by formula (X-1) to 1,3-dichloro-5,5-dimethylhydantoin is 1:(0.5-0.7);
optionally, the molar ratio of the compound represented by formula (X-1) to N-chlorosuccinimide is 1:(1.1-1.6);
optionally, the chlorination reaction is carried out at a temperature of 55-60°C.

10. A method for preparing compound 6, wherein it comprises performing an oxidation reaction and deprotection reaction on the compound represented by formula (Y-2) to obtain compound 6,

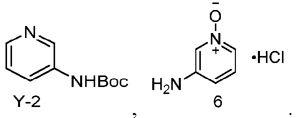

11. The method according to claim 10, wherein the compound represented by formula (Y-2) is obtained by performing an acylation reaction with the compound represented by formula (Y-1),
